(19) Europäisches Patentamt
European Patent Office
Office européen des brevets

(11) Veröffentlichungsnummer: **0 051 177**
**A2**

(12) # EUROPÄISCHE PATENTANMELDUNG

(21) Anmeldenummer: 81108314.6

(22) Anmeldetag: 14.10.81

(51) Int. Cl.³: C 07 D 491/04
A 61 K 31/505
//(C07D491/04, 311/00, 239/00)

(30) Priorität: 17.10.80 CH 7774/80

(43) Veröffentlichungstag der Anmeldung: 12.05.82 Patentblatt 82/19

(84) Benannte Vertragsstaaten: AT BE CH DE FR IT LI LU NL SE

(71) Anmelder: CIBA-GEIGY AG
Patentabteilung Postfach
CH-4002 Basel(CH)

(72) Erfinder: Haas, Georges, Dr.
Im Rehwechsel 24
CH-4102 Binningen(CH)

(72) Erfinder: Sele, Alex
Langmattstrasse 14
CH-4132 Muttenz(CH)

(72) Erfinder: Jaeggi, Knut A., Dr.
General Guisan-Strasse 44
CH-4054 Basel(CH)

(72) Erfinder: Eichenberger, Kurt, Dr.
Neubergweg 36
CH-4106 Therwil(CH)

(74) Vertreter: Zumstein sen., Fritz, Dr. et al,
Bräuhausstrasse 4
D-8000 München 2(DE)

(54) **Pharmazeutische Präparate enthaltend substituierte Äther-Verbindungen, ihre Verwendung, neue substituierte Äther-Verbindungen, sowie Verfahren zu ihrer Herstellung.**

(57) Die Erfindung betrifft pharmazeutische Präparate enthaltend neue substituierte Aether der Formel I

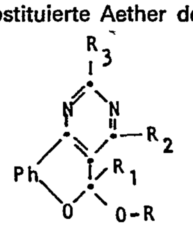

(I),

worin Ph einen gegebenenfalls substituierten 1,2-Phenylenrest bedeutet, R für einen gegebenenfalls durch einen $Ph_1$-Rest substituierten niederaliphatischen Kohlenwasserstoffrest, wobei $Ph_1$ ein gegebenenfalls substituierter Phenylrest ist, einen 3 bis 7 Kohlenstoffatome enthaltenden Cycloalkylrest, einen durch einen heterocyclischen Rest aromatischen Charakters substituierten Niederalkylrest oder einen Rest der Formel $(CH_2CH_2O)_n$-$CH_2CH_2OR_4$ steht, worin n 0, 1 oder 2 ist und $R_4$ Wasserstoff oder Niederalkyl bedeutet, jedes der Symbole $R_1$ und $R_2$ für Wasserstoff oder Niederalkyl steht, $R_3$ einen gegebenenfalls substituierten Phenylrest $Ph_1$, einen gegebenenfalls durch $Ph_1$ substituierten Niederalkylrest, einen 3 bis 7 Kohlenstoffatome enthaltenden Cycloalkylrest, einen Cycloalkyl-$Ph_1$-Rest, worin Cycloalkyl und $Ph_1$ die oben angegebenen Bedeutungen haben, oder einen heterocyclischen Rest aromatischen Charakters bedeutet, und therapeutisch verwendbare Salze dieser Verbindungen, mit lipidsenkenden, antiarteriosklerotischen und analgetischen Wirkungen. Ebenfalls umfasst sind neue substituierte Aether der Formel I, und ihre Salze, mit Ausnahme der Verbindung der Formel I, worin Ph unsubstituiertes 1,2-Phenylen bedeutet, R für Aethyl steht, jedes der Symbole $R_1$ und $R_3$ Methyl bedeutet, $R_2$ für Wasserstoff steht, und ihres Perchlorats. Die neuen Verbindungen können z.B. durch Verätherung einer der Formel I entsprechenden Verbindung, worin R für Wasserstoff steht.

EP 0 051 177 A2

Croydon Printing Company Ltd.

0051177



CIBA-GEIGY AG
Basel (Schweiz)

4-13107/+

# Pharmazeutische Präparate enthaltend substituierte Aether-Verbindungen, ihre Verwendung, neue substituierte Aether-Verbindungen, sowie Verfahren zu ihrer Herstellung

Die Erfindung betrifft pharmazeutische Präparate enthaltend substituierte Aether von 5-Hydroxy-5H-[1]benzopyrano[4,3-d]pyrimidinen und ihre therapeutische Verwendung.

In Liebigs Ann. Chem. 1976, p. 1663-1673 wird von U. Petersen und H. Heitzer die Umsetzung von 4-Oxo-4H-chromen-3-carbaldehyden mit Amidinen beschrieben, wobei 5-Hydroxy-5H-[1]benzopyrano[4,3-d]pyrimidinverbindungen erhalten werden. Weiter ist in Arch. Pharm. (Weinheim), 310, S. 559-563 (1977), von W. Löwe das Perchlorat des 5-Aethoxy-2,5-dimethyl-5H-[1]benzopyrano[4,3-d]pyrimidins offenbart. Eine pharmakologische Wirkung dieser Verbindungen ist jedoch nicht beschrieben.

Es ist nun überraschend gefunden worden, dass die substituierten Aether von 5-Hydroxy-5H-[1]benzopyrano[4,3-d]pyrimidinen wertvolle pharmakologische Wirkungen aufweisen.

Die Erfindung betrifft daher insbesondere pharmazeutische Präparate enthaltend substituierte Aether von 5-Hydroxy-5H-[1]benzopyrano[4,3-d]pyrimidinen der allgemeinen Formel I

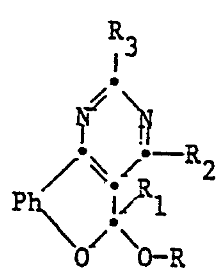

(I),

worin Ph einen gegebenenfalls substituierten 1,2-Phenylenrest bedeutet, R für einen gegebenenfalls durch einen $Ph_1$-Rest substituierten niederaliphatischen Kohlenwasserstoffrest, wobei $Ph_1$ ein gegebenenfalls substituierter Phenylrest ist, einen 3 bis 7 Kohlenstoffatome enthaltenden Cycloalkylrest, einen durch einen heterocyclischen Rest aromatischen Charakters substituierten Niederalkylrest oder einen Rest der Formel $(CH_2CH_2O)_n-CH_2CH_2OR_4$ steht, worin n 0, 1 oder 2 ist und $R_4$ Wasserstoff oder Niederalkyl bedeutet, jedes der Symbole $R_1$ und $R_2$ für Wasserstoff oder Niederalkyl steht, $R_3$ einen gegebenenfalls substituierten Phenylrest $Ph_1$, einen gegebenenfalls durch $Ph_1$ substituierten Niederalkylrest, einen 3 bis 7 Kohlenstoffatome enthaltenden Cycloalkylrest, einen Cycloalkyl-$Ph_1$-Rest, worin Cycloalkyl und $Ph_1$ die oben angegebenen Bedeutungen haben, oder einen heterocyclischen Rest aromatischen Charakters bedeutet, und therapeutisch verwendbare Salze dieser Verbindungen.

Der Ausdruck "nieder" definiert in den oben oder nachfolgend genannten organischen Resten oder Verbindungen solche mit höchstens 7, vorzugsweise 4, insbesondere 1 oder 2 Kohlenstoffatomen.

Vorstehend und nachfolgend können die Allgemeinbegriffe folgende Bedeutung haben:

Ein gegebenenfalls substituierter 1,2-Phenylenrest Ph kann z.B. durch einen, zwei, oder drei gleiche oder verschiedene Substituenten substituiert sein. Solche Substituenten sind z.B. Hydroxy, Niederalkyl, Hydroxy-niederalkyl, Niederalkoxy, Niederalkoxycarbonyl, Niederalkoxycarbonylniederalkyl, Halogen, Halogen-niederalkyl, Trifluormethyl, Nitro oder Amino und überdies in zwei benachbarten Stellungen ein anellierter 1,2-Cycloalkylen- oder 1,2-Phenylenrest.

Ein gegebenenenfalls substituierter Phenylrest $Ph_1$ kann durch einen zwei oder drei gleiche oder verschiedene Substituenten substituiert sein. Solche Substituenten sind z.B. Hydroxy, Niederalkyl, Hydroxy-niederalkyl, Niederalkoxy, Niederalkoxycarbonyl, Niederalkoxycarbonylniederalkyl, Halogen, Halogen-niederalkyl, Trifluoromethyl, Nitro oder Amino.

Ein niederaliphatischer Kohlenwasserstoffrest ist ein Niederalkyl-, Niederalkenyl- oder Niederalkinylrest.

Niederalkylgruppen sind z.B. Methyl- sowie Aethyl-, n-Propyl-, Isopropyl-, n-Butyl-, Isobutyl-, sek. Butyl, tert. Butyl-, n-Pentyl-, Isopentyl-, Neopentyl-, n-Hexyl-, Isohexyl oder n-Heptylgruppen.

In den Hydroxy-niederalkylgruppen sind z.B. die vorher genannten Niederalkylgruppen an beliebiger Stelle durch Hydroxy substituiert.

Niederalkenylgruppen sind z.B. die Allyl- oder die 2-Methylallylgruppe und Niederalkinylgruppen vorzugsweise Propargylgruppen.

Eine Niederalkoxycarbonylgruppe ist z.B. die Methoxycarbonyl- oder Aethoxycarbonylgruppe.

Eine Niederalkoxycarbonyl-niederalkylgruppe ist z.B. die Methoxycarbonyl-methylgruppe oder Aethoxycarbonyl-methylgruppe.

Eine Cycloalkylgruppe ist z.B. eine Cyclopropyl-, Cyclobutyl-, Cyclopentyl-, Cyclohexyl- oder Cycloheptylgruppe.

Ein heterocyclischer Rest aromatischen Charakters ist vorzugsweise monocyclisch, wie Pyrryl, z.B. 2-Pyrryl oder 3-Pyrryl, Pyridyl, z.B. 2-, 3- oder 4-Pyridyl, ferner Thienyl, z.B. 2- oder 3-Thienyl, oder Furyl, z.B. 2-Furyl.

Niederalkoxy ist z.B. Methoxy, Aethoxy, n-Propoxy, Isopropoxy, n-Butoxy oder n-Pentyloxy.

Halogenatome sind in erster Linie Fluor-, Chlor- oder Bromatome, können jedoch auch Jodatome sein.

Halogen-niederalkylgruppen weisen das Halogenatom an beliebiger Stelle eines Niederalkylrestes auf.

Die basischen Verbindungen der allgemeinen Formel I bilden Säureadditionssalze. In den Präparaten der Erfindung werden therapeutisch verwendbare, nicht toxische, Säureadditionssalze eingesetzt. Für ihre Herstellung werden z.B. anorganische Säuren, z.B. Chlorwasserstoff- oder Bromwasserstoffsäure, oder Schwefel-, Phosphor-, Salpeter- oder Perchlorsäure; oder organische Säuren, wie Carbon- oder Sulfonsäuren, z.B. Ameisen-, Essig-, Propion-, Bernstein-, Glykol, Milch-, Aepfel-, Wein-, Zitronen-, Malein-, Hydroxymalein-, Brenztrauben-, Phenylessig-, Benzoe-, 4-Aminobenzoe-, Anthranil-, 4-Hydroxybenzoe-, Salicyl-, Pamoa-, Nikotin-, Methansulfon-, Aethansulfon-, Hydroxyäthansulfon-, Aethylensulfon-, Benzolsulfon-, Halogenbenzolsulfon-, Toluolsulfon-, Naphthalinsulfon-, Sulfanil- oder Cyclohexylsulfaminsäure; oder die Ascorbinsäure verwendet. Andererseits bilden Verbindungen, welche mindestens eine phenolische Hydroxygruppe aufweisen, Metallsalze, insbesondere Alkalimetallsalze oder Erdalkalimetallsalze, z.B. Natrium- oder Kaliumsalze. In den Präparaten werden therapeutisch verwendbare , nicht-toxische Metallsalze verwendet.

Die in den Präparaten der Erfindung verwendeten Verbindungen zeigen wertvolle pharmakologische Eigenschaften, z.B. hypolipidämische Wirkungen mit günstiger Beeinflussung des Lipoproteinspektrums, ferner, insbesondere Verbindungen der Formel I, worin $R_3$ für einen heterocyclischen Rest aromatischen Charakters, in erster Linie Pyridyl steht, analgetische Effekte. Diese pharmakologischen Eigenschaften können in Tierversuchen, vorzugsweise an Säugetieren, z.B. Ratten nachgewiesen werden. Hierbei können die Verbindungen enteral oder parenteral, vorzugsweise oral, subkutan oder intraperitoneal in einem Dosisbereich von 1 bis 100 mg/kg/Tag verabreicht werden. Beispielsweise wird die Senkung von "low und very low-density"-Lipoproteinen (LDL und VLDL), sowie die Senkung von Cholesterin und Triglyceriden im Serum wie folgt bestimmt: Männlichen Ratten verabreicht man an 3 Tagen einmal täglich

50 mg Wirkstoff peroral. Am 4. Tag werden die Tiere 2-mal behandelt. Am 5. Tag wird Serum gewonnen und die Lipoproteine werden mit einer Ultrazentrifuge aufgetrennt. Im Serum und in den isolierten Lipoproteinfraktionen werden das Cholesterin und die Triglyceride bestimmt. [K.R. Müller und R.G. Cortesi, Artery 4 (6): 564-577 (1978)].

Die analgetische Wirkung lässt sich z.B. an der Ratte bei oraler Verabreichung von Dosen ab 1 mg/kg nachweisen.

Die Präparate mit den oben genannten Verbindungen können daher als Lipidsenker bzw. Antiarteriosklerotika oder Analgetika verwendet werden.

Bevorzugte pharmazeutische Präparate enthalten Verbindungen der Formel I, worin Ph einen 1,2 Phenylenrest bedeutet, der gegebenenfalls durch Hydroxy, Niederalkyl, Hydroxy-niederalkyl, Niederalkoxy, Niederalkoxycarbonyl, Niederalkoxycarbonylniederalkyl, Halogen, Halogenniederalkyl, Trifluormethyl, Nitro oder Amino substituiert sein kann, R für Niederalkyl, Niederalkenyl, Niederalkinyl, Cycloalkyl mit 3 bis 7 Kohlenstoffatomen, $Ph_1$-Niederalkyl, worin der Phenylrest $Ph_1$ analog dem oben genannten Rest Ph substituiert sein kann, oder für einen durch einen Pyrryl-, Pyridyl- oder Thienylrest substituierten Niederalkylrest steht, jedes der Symbole $R_1$ und $R_2$ Wasserstoff oder Niederalkyl bedeutet, $R_3$ für einen oben definierten Phenylrest $Ph_1$, einen gegebenenfalls durch einen solchen Phenylrest $Ph_1$ substituierten Niederalkylrest oder für einen Pyrryl-, Pyridyl- oder Thienylrest steht, und therapeutisch verwendbare Salze dieser Verbindungen.

Besonders bevorzugte pharmazeutische Präparate enthalten Verbindungen der Formel I, worin Ph einen 1,2-Phenylenrest bedeutet, der gegebenenfalls durch Hydroxy, Niederalkyl, Niederalkoxy, Niederalkoxycarbonylniederalkyl, Halogen oder Trifluormethyl substituiert sein kann, R für Niederalkyl oder $Ph_1$-Niederalkyl steht, worin der Phenylrest $Ph_1$ analog dem oben genannten Rest Ph substituiert sein kann,

jedes der Symbole $R_1$ und $R_2$ Niederalkyl, vorzugsweise Wasserstoff bedeutet, $R_3$ für einen wie oben definierten Phenylrest $Ph_1$, einen gegebenenfalls durch einen solchen Phenylrest $Ph_1$ substituierten Niederalkylrest oder für Pyridyl steht, und therapeutisch verwendbare Salze dieser Verbindungen.

Hervorzuheben sind die pharmazeutischen Präparate, welche die Verbindungen der Formel I enthalten, worin Ph den 1,2-Phenylenrest bedeutet, R für Niederalkyl oder Phenyl-niederalkyl steht, jedes der Symbole $R_1$ und $R_2$ Wasserstoff bedeutet, $R_3$ für Niederalkyl, Phenyl, Niederalkoxy-phenyl, Phenyl-niederalkyl oder Pyridyl steht, und therapeutisch verwendbare Salze dieser Verbindungen.

Besonders hervorzuheben sind pharmazeutische Präparate, welche die Verbindungen der Formel I enthalten, worin Ph den 1,2-Phenylenrest bedeutet, R für Niederalkyl steht, jedes der Symbole $R_1$ und $R_2$ Wasserstoff bedeutet, $R_3$ für den gegebenenfalls durch Niederalkoxy substituierten Phenylrest steht, und therapeutisch verwendbare Salze dieser Verbindungen.

Ebenfalls betrifft die Erfindung neue substituierte Aether von 5-Hydroxy-5H-[1]benzopyrano[4,3-d]pyrimidinen und Verfahren zu ihrer Herstellung.

Die Erfindung betrifft insbesondere neue substituierte Aether von 5-Hydroxy-5H-[1]benzopyrano[4,3-d]pyrimidinen der oben angegebenen allgemeinen Formel I, worin PH einen gegebenenfalls substituierten 1,2-Phenylenrest bedeutet, R für einen gegebenenfalls durch einen $Ph_1$-Rest substituierten niederaliphatischen Kohlenwasserstoffrest, wobei $Ph_1$ ein gegebenenfalls substituierter Phenylrest ist,
einen 3 bis 7 Kohlenstoffatome enthaltenden
Cycloalkylrest, einen durch einen heterocyclischen Rest

aromatischen Charakters substituierten Niederalkylrest oder einen Rest der Formel $-(CH_2CH_2O)_n-CH_2CH_2OR_4$ steht, worin n 0, 1 oder 2 ist und $R_4$ Wasserstoff oder Niederalkyl bedeutet, jedes der Symbole $R_1$ und $R_2$ für Wasserstoff oder Niederalkyl steht, $R_3$ einen gegebenenfalls substituierten Phenylrest $Ph_1$, einen gegebenenfalls durch $Ph_1$ substituierten Niederalkylrest, einen 3 bis 7 Kohlenstoffatome enthaltenden Cycloalkylrest, einen Cycloalkyl-$Ph_1$-Rest, worin Cycloalkyl und $Ph_1$ die oben angegebenen Bedeutungen haben, oder einen heterocyclischen Rest aromatischen Charakters bedeutet, und ihre Salze, insbesondere therapeutisch verwendbare Salze dieser Verbindungen, mit Ausnahme der Verbindung der Formel I, worin Ph unsubstituiertes 1,2-Phenylen bedeutet, R für Aethyl steht, jedes der Symbole $R_1$ und $R_3$ Methyl bedeutet, $R_2$ für Wasserstoff steht, und ihres Perchlorats.

Die Bedeutung der Allgemeinbegriffe ist bereits oben angegeben worden.

Die freien basischen Verbindungen der Formel I können in entsprechende Säureadditionssalze mit anorganischen oder organischen Säuren, insbesondere mit den oben genannten, welche therapeutisch verwendbare Säureadditionssalze ergeben, übergeführt werden. Andererseits können Verbindungen mit einer freien phenolischen Hydroxygruppe z.B. mit Alkalimetallhydroxydlösungen in ihre Alkalimetallsalze umgewandelt werden.

Die neuen Verbindungen zeigen die oben genannten pharmakologischen Wirkungen. Sie können daher als Lipidsenker bzw. Antiarteriosklerotika oder Analgetika verwendet werden. Sie können auch als Zwischenprodukte zur Herstellung von anderen wertvollen, insbesondere von pharmakologisch wirksamen Verbindungen oder Präparaten eingesetzt werden.

Bevorzugte Verbindungen sind diejenigen der Formel I, worin Ph einen 1,2-Phenylenrest bedetet, der gegebenenfalls durch Hydroxy, Niederalkyl, Hydroxy-niederalkyl, Niederalkoxy, Niederalkoxycarbonyl, Niederalkoxycarbonylniederalkyl, Halogen, Halogen-niederalkyl, Trifluormethyl,

Nitro oder Amino substituiert sein kann,
R für Niederalkyl, Niederalkenyl, Niederalkinyl, Cycloalkyl mit 3 bis 7 Kohlenstoffatomen, $Ph_1$-Niederalkyl, worin der Phenylrest $Ph_1$ analog dem oben genannten Rest Ph substituiert sein kann, oder einen durch einen Pyrryl-, Pyridyl- oder Thienylrest substituierten Niederalkylrest steht, jedes der Symbole $R_1$ und $R_2$ Wasserstoff oder Niederalkyl bedeutet, $R_3$ für einen oben definierten Phenylrest $Ph_1$, einen gegebenenfalls durch einen solchen Phenylrest $Ph_1$ substituierten Niederalkylrest oder für einen Pyrryl-, Pyridyl- oder Thienylrest steht, und Salze, insbesondere therapeutisch verwendbare Salze dieser Verbindungen, mit Ausnahme der Verbindung der Formel I, worin Ph unsubstituiertes 1,2-Phenylen bedeutet, R für Aethyl steht, jedes der Symbole $R_1$ und $R_3$ Methyl bedeutet, $R_2$ für Wasserstoff steht, und ihres Perchlorats.

Besonders bevorzugt sind Verbindungen der Formel I, worin Ph einen 1,2-Phenylenrest bedeutet, der gegebenenfalls durch Hydroxy, Niederalkyl, Niederalkoxy, Niederalkoxycarbonylniederalkyl, Halogen oder Trifluormethyl substituiert sein kann, R für Niederalkyl oder $Ph_1$-Niederalkyl steht, worin der Phenylrest $Ph_1$ analog dem oben genannten Rest Ph substituiert sein kann, jedes der Symbole $R_1$ und $R_2$ Niederalkyl, vorzugsweise Wasserstoff bedeutet, $R_3$ für einen wie oben definierten Phenylrest $Ph_1$, einen gegebenenfalls durch einen solchen Phenylrest $Ph_1$ substituierten Niederalkylrest oder für Pyridyl steht, und Salze, insbesondere therapeutisch verwendbare Salze dieser Verbindungen, mit Ausnahme der Verbindung der Formel I, worin Ph unsubstituiertes 1,2-Phenylen bedeutet, R für Aethyl steht, jedes der Symbole $R_1$ und $R_3$ Methyl bedeutet, $R_2$ für Wasserstoff steht, und ihres Perchlorats.

Hervorzuheben sind die Verbindungen der Formel I, worin Ph den 1,2-Phenylenrest bedeutet, R für Niederalkyl oder Phenyl-niederalkyl steht, jedes der Symbole $R_1$ und $R_2$ Wasserstoff bedeutet, $R_3$ für Niederalkyl, Phenyl, Niederalkoxy-phenyl, Phenyl-niederalkyl oder Pyridyl

steht, und Salze, insbesondere therapeutisch verwendbare Salze dieser Verbindungen.

Besonders hervorzuheben sind die Verbindungen der Formel I, worin Ph den 1,2-Phenylenrest bedeutet, R für Niederalkyl steht, jedes der Symbole $R_1$ und $R_2$ Wasserstoff bedeutet, $R_3$ für den gegebenenfalls durch Niederalkoxy substituierten Phenylrest steht, und Salze, insbesondere therapeutisch verwendbare Salze dieser Verbindungen.

Insbesondere betrifft die Erfindung die neuen, in den Beispielen beschriebenen Verbindungen und ihre Salze, insbesondere therapeutisch verwendbare Salze dieser Verbindungen.

Die Verbindungen der vorliegenden Erfindung werden nach an sich bekannten Verfahren erhalten.

Die neuen Verbindungen der Formel I werden beispielsweise hergestellt, indem man

a) eine Verbindung der allgemeinen Formel II mit einer Verbindung der allgemeinen Formel III

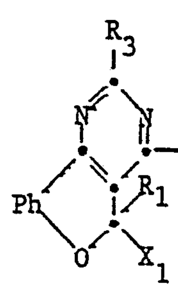 (II) mit $X_2$-R (III)

veräthert, worin einer der Reste $X_1$ und $X_2$ eine freie, metallisierte oder reaktionsfähige veresterte Hydroxygruppe bedeutet und der andere für eine freie, reaktionsfähige veresterte bzw. metallisierte Hydroxygruppe steht, oder $X_2$ gemeinsam mit dem Rest R in der Verbindung $X_2$-R eine den Rest R einführende Verbindung darstellt, wenn $X_1$ eine freie Hydroxygruppe bedeutet, oder

b) eine Verbindung der allgemeinen Formel IV

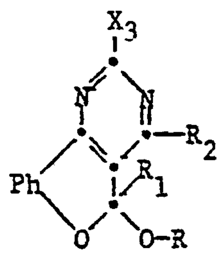

(IV),

worin $X_3$
eine abspaltbare Gruppe bedeutet, mit einer metallorganischen Verbindung der Formel $R_3$-M, worin M für eine metallische Gruppierung steht, oder mit einem $R_3$-Halogenid in Anwesenheit eines Metalls, kondensiert, und, wenn erwünscht, eine erhaltene Verbindung der Formel I in eine andere erfindungsgemässe Verbindung umwandelt, und/oder, wenn erwünscht, eine erhaltene freie Verbindung in ein Salz oder ein Salz in die freie Verbindung oder in ein anderes Salz überführt, und/oder wenn erwünscht, ein erhaltenes Gemisch von Isomeren oder Razematen in die einzelnen Isomeren oder Razemate auftrennt, und/oder, wenn erwünscht, erhaltene Razemate in die optischen Antipoden aufspaltet.

Falls $X_2$-R eine den Rest R einführende Verbindung bedeutet, so kann sie eine entsprechende Diazoverbindung, ein dem Alkohol ROH entsprechendes Acetal oder einen entsprechenden Orthoester, ein entsprechendes Oxonium-, Carbenium- oder Haloniumsalz oder eine entsprechende Triazenverbindung darstellen.

Bedeutet $R_1$ in der Formel II Wasserstoff und $X_1$ die freie Hydroxygruppe, so kann dieser Ausgangsstoff auch in der tautomeren Form der Formel IIa

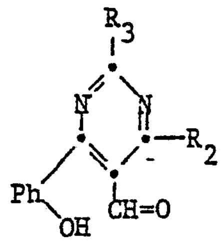

(IIa)

vorliegen.

Stehen die beiden Substituenten $X_1$ und $X_2$ in den Ausgangsstoffen der Formeln II und III jeweils für eine freie Hydroxygruppe, so wird die Reaktion in Gegenwart von Protonendonatoren, d.h. unter Säurekatalyse durchgeführt. Als Protonendonatoren werden in erster Linie starke anorganische Säuren oder organische Sulfonsäuren, beispielsweise Mineralsäuren, wie Halogenwasserstoffsäuren, z.B. Chlorwasserstoffsäure, ferner Schwefelsäure, oder z.B. die p-Toluolsulfonsäure, aber auch Lewis Säuren, wie Halogenide des Bors, Aluminiums oder Zinks, z.B. Bortrifluorid, Aluminiumchlorid oder Zinkchlorid, verwendet. Die Verätherung wird vorzugsweise ohne Lösungsmittelzusatz in der entsprechenden alkoholischen Lösung, d.h. in einem Alkohol der Formel ROH durchgeführt, sofern dieser bei Ausführungstemperatur in flüssigem Zustand vorliegt.

Ist einer der Reste $X_1$ und $X_2$ eine freie oder metallisierte, vorzugsweise eine durch ein Alkalimetallatom metallisierte Hydroxygruppe, z.B. ONa, so liegt der andere Rest in Form einer reaktionsfähigen veresterten Hydroxygruppe vor. Eine reaktionsfähige veresterte Hydroxygruppe ist vorzugsweise eine durch eine starke Mineral- oder Sulfonsäure, wie eine Halogenwasserstoff-, Schwefel-, Niederalkansulfon- oder Benzolsulfonsäure, z.B. Salz-, Bromwasserstoff-, Methansulfon-, Trifluormethansulfon-, Benzolsulfon- oder p-Toluolsulfonsäure veresterte Hydroxygruppe. Solche Ester sind u.a. Niederalkylhalogenide, Diniederalkylsulfate, wie Dimethylsulfat, ferner Fluorsulfonsäureester, wie Niederalkylester, z.B. Fluorsulfonsäuremethylester, oder gegebenenfalls Halogen-substituierte Methansulfonsäure-niederalkylester, z.B. Trifluormethansulfonsäuremethylester. Die Hydroxygruppe des Ausgangsstoffes der Formel II bzw. III kann aber auch z.B. durch eine Niederalkansäure, wie Essigsäure oder Propionsäure verestert sein. Wenn einer der Reste $X_1$ oder $X_2$ die freie Hydroxygruppe bedeutet, wird die Verätherung in Gegenwart von basischen Kondensationmitteln, welche die gebildeten Säuren binden, durchgeführt. Solche Mittel sind Erdalkali- oder Alkalimetall-carbonate oder -hydrogencarbonate, z.B. Calcium- oder Natrium-carbonate oder -hydrogencarbonate, oder tertiäre Amine, z.B. Tri-niederalkylamine, Pyridine bzw. niederalkylierte Pyridine. Wird der eine Ausgangsstoff in Form der metallisierten Verbindung (z.B. $X_1$ = ONa) eingesetzt, so arbeitet man unter neutralen Reaktionsbedingungen. Liegt schliesslich z.B. die Hydroxygruppe $X_1$ als eine durch Niederalkansäure, z.B. Essigsäure veresterte Hydroxygruppe vor, so kann die Reaktion mit einem entsprechenden Alkohol ROH in saurem Medium, vorzugsweise in Gegenwart einer Mineralsäure, z.B. Halogenwasserstoffsäure, wie Chlorwasserstoffsäure, durchgeführt werden. Die Reaktionen werden, falls nötig, unter Zusatz eines inerten Lösungsmittels, wie eines gegebenenfalls halogenierten, wie chlorierten, aliphatischen, cycloaliphatischen oder aromatischen Kohlenwasserstoffs, z.B. Methylenchlorid, eines Aethers, wie Dioxan oder Tetrahydrofuran, oder eines Gemisches dieser Lösungsmittel, vorgenommen.

Durch Phasentransfer-Katalyse [siehe Dehmlow, Angewandte Chemie, Bd. 5, S. 187 (1974)] kann die oben beschriebene Verätherungsreaktion wesentlich beschleunigt werden. Als Phasentransfer-Katalysatoren können quartäre Phosphoniumsalze und insbesondere quartäre Ammoniumsalze, wie gegebenenfalls substituierte Tetraalkylammoniumhalogenide, z.B. Tetrabutylammoniumchlorid, -bromid oder -jodid, oder auch Benzyl-triäthylammoniumchlorid, in katalytischen oder bis zu äquimolaren Mengen verwendet werden. Als organische Phase kann irgendeines mit Wasser nicht mischbares Lösungsmittel dienen, beispielsweise einer der gegebenenfalls halogenierten, wie chlorierten niederaliphatischen, cycloaliphatischen oder aromatischen Kohlenwasserstoffe, wie Tri- oder Tetrachloräthylen, Tetrachloräthan, Tetrachlorkohlenstoff, Chlorbenzol, Toluol oder Xylol. Als Kondensationsmittel geeignete Alkalimetall-carbonate oder -hydrogencarbonate sind z.B. Kalium- oder Natriumcarbonat oder -hydrogencarbonat, Alkalimetallphosphate, z.B. Kaliumphosphat, und Alkalimetallhydroxide, z.B. Natriumhydroxid.

Ausgangsstoffe der Formel II, worin $X_1$ für die freie Hydroxygruppe steht, können, wie bereits oben gesagt, auch durch Umsetzung mit entsprechenden Diazoverbindungen veräthert werden. Solche Verbindungen sind z.B. Diazoniederalkane, wie Diazomethan, Diazoäthan, Diazo-n-butan, aber auch $Ph_1$-Diazoniederalkane, z.B. Phenyl-diazomethan. Diese Reagentien werden in Gegenwart eines geeigneten inerten Lösungsmittels, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z.B. Methylenchlorid, oder eines Aethers, wie eines Diniederalkyläthers, z.B. Diäthyläther, oder eines cyclischen Aethers, z.B. Tetrahydrofuran oder Dioxan, oder eines Lösungsmittelgemisches, und, je nach Diazoreagens, unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre, zur Anwendung gebracht.

Wenn $X_1$ Hydroxy bedeutet, sind weitere Verätherungsmittel geeignete Acetal-Verbindungen, z.B. gem-Di-niederalkoxy-niederalkane, wie 2,2-Dimethoxy-propan, die in Gegenwart von starken organischen Sulfonsäuren, wie p-Toluolsulfonsäure, und eines geeigneten Lösungsmittels, wie eines Diniederalkyl- oder Niederalkylensulfoxyds, z.B. Dimethylsulfoxyd, zur Anwendung gelangen, oder geeignete Orthoester, z.B. Orthoameisensäuretriniederalkylester, z.B. Orthoameisensäuretriäthylester, die in Gegenwart einer starken Mineralsäure, z.B. Schwefelsäure, oder einer starken organischen Sulfonsäure, wie p-Toluolsulfonsäure, und eines geeigneten Lösungsmittels, wie eines Aethers, z.B. Dioxan, verwendet werden.

Steht $X_1$ für die freie Hydroxygruppe, sind weitere Verätherungsmittel entsprechende trisubstituierte Oxoniumsalze (sogenannte Meerweinsalze), oder disubstituierte Carbenium- oder Haloniumsalze, worin die Substituenten die veräthernden Reste R sind, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate oder Hexachlorantimonate. Solche Reagentien sind z.B. Trimethyloxonium- oder Triäthyloxonium-hexafluorantimonat, -hexachlorantimonat, -hexafluorphosphat oder -tetrafluorborat, Dimethoxycarbeniumhexafluorphosphat oder Dimethylbromoniumhexafluorantimonat. Man verwendet diese Verätherungsmittel vorzugsweise in einem inerten Lösungsmittel, wie einem Aether oder einem halogenierten Kohlenwasserstoff, z.B. Diäthyläther, Tetrahydrofuran oder Methylenchlorid, oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-äthyl-amin, und unter Kühlen, bei Raumtemperatur oder unter leichtem Erwärmen, z.B. bei etwa -20°C bis etwa 50°C, wenn notwendig, in einem geschlossenen Gefäss und/oder in einer Inertgas-, z.B. Stickstoffatomosphäre.

Ist der Substituent $X_1$ des Ausgangsstoffes der Formel II eine freie Hydroxygruppe, so sind schliesslich weitere Verätherungsmittel entsprechende 1-substituierte 3-Aryltriazenverbindungen, worin der Substituent den veräthernden Rest R, und Aryl vorzugsweise gegebenenfalls substituiertes Phenyl, z.B. Niederalkylphenyl, wie 4-Methylphenyl, bedeutet. Solche Triazenverbindungen sind 3-Aryl-1-niederalkyltriazene, z.B. 3-(4-Methylphenyl)-1-methyl-triazen, 3-(4-Methylphenyl)-1-äthyl-triazen oder 3-(4-methylphenyl)-1-isopropyl-triazen. Diese Reagentien werden üblicherweise in Gegenwart von inerten Lösungsmitteln, wie gegebenenfalls halogenierten Kohlenwasserstoffen oder Aethern, z.B. Benzol, oder Lösungsmittelgemischen, und unter Kühlen, bei Raumtemperatur und vorzugsweise bei erhöhter Temperatur, z.B. bei etwa 20°C bis etwa 100°C, wenn notwendig, in einem geschlossenen Gefäss und/oder in einer Inertgas-, z.B. Stickstoffatmosphäre verwendet.

In den Ausgangsstoffen der Verfahrensvariante b) ist die abspaltbare Gruppe $X_3$ z.B. der Rest einer starken anorganischen Säure, wie einer Halogenwasserstoffsäure, z.B. Chlorwasserstoff-, Bromwasserstoff- oder Jodwasserstoffsäure, d.h. ein Chlor-, Brom- oder Jodatom, oder der Schwefelsäure, oder einer organischen Sulfonsäure, vorzugsweise Niederalkansulfonsäure, z.B. Methylsulfonyl oder Aethylsulfonyl. In den Verbindungen der Formel $R_3$-M ist die metallische Gruppierung M vorzugsweise en Erdalkali- oder Alkalimetallatom, z.B. Calcium, Magnesium Natrium, Kalium oder Lithiumatom. In der Kondensationsreaktion verwendete Metalle sind in erster Linie Erdalkali-, insbesondere jedoch Alkalimetalle, z.B. Natrium oder Kalium. Es können aber auch Schwermetalle, wie z.B. Kupfer verwendet werden. Die Reaktion wird vorzugsweise in Gegenwart von inerten Lösungsmitteln, in erster Linie in aromatischen Kohlenwasserstoffen, z.B. Benzol oder Toluol oder in einem Aether, z.B. Diäthyläther, durchgeführt.

Bei Anwesenheit einer freien phenolischen Hydroxygruppe wählt man zur Ausführung der verfahrensgemässen Verätherung solche Mittel und/oder Bedingungen, die selektiv nur die Hydroxygruppe in 5-Stellung des Ausgangsstoffes veräthern, wie z.B. die Behandlung unter relativ milden Bedingungen mit dem betreffenden Alkohol in saurem Medium.

In erhaltenen Verbindungen kann man im Rahmen der Definition der Endprodukte Substituenten abwandeln. So kann man in einem Produkt der Formel I den Substituenten R z.B. durch Behandlung mit einem anderen Alkohol der Formel ROH, gegebenenfalls in Gegenwart einer Säure, gegen einen anderen Substituenten R austauschen. Enthält ein Produkt eine phenolische Hydroxygruppe, so kann diese in bekannter Weise, z.B. durch Umsetzung mit einem Diazoniederalkan oder durch Reaktion mit einem Niederalkylhalogenid wie Jodid oder Bromid, z.B. in Gegenwart von Silberoxyd oder Silbercarbonat, in eine Niederalkoxygruppe umgewandelt werden. Ist im 1,2-Phenylenrest Ph oder im Phenylrest $Ph_1$ eine Nitrogruppe vorhanden, so kann diese in bekannter Weise, z.B. durch Behandlung mit einem Alkalimetall-niederalkanolat, wie Natrium-methylat oder Natrium-äthylat, vorzugsweise in einem wasserfreien Niederalkanol, z.B. Methanol oder Aethanol, durch eine Niederalkoxy-, wie Methoxy- oder

Aethoxygruppe ersetzt werden. Eine Nitrogruppe kann auch, in bekannter Weise, z.B. mit katalytisch aktiviertem Wasserstoff, in die Aminogruppe übergeführt werden.

Säureadditionssalze von Verbindungen der Formel I erhält man in üblicher Weise, z.B. durch Behandlung mit einer Säure oder einem geeigneten Anionenaustauscher. Die erhaltenen Salze können in an sich bekannter Weise, z.B. durch Behandeln mit einem geeigneten basischen Mittel, z.B. einem Metallhydroxyd, Ammoniak oder einem Hydroxylionaustauscher, in die freien Verbindungen übergeführt werden. Andererseits können Verbindungen mit einer phenolischen Hydroxygruppe durch Behandlung z.B. mit einem Alkalimetallhydroxyd in an sich bekannter Weise in ein Alkalimetallsalz umgewandelt werden. Durch Behandlung mit einer Säure können die freien Verbindungen erhalten werden.

Bevorzugt sind die oben genannten therapeutisch verwendbaren Salze. Diese oder andere Salze, z.B. die Pikrate, können auch in der Reinigung von freien Basen verwendet werden. Die Basen werden in ihre Salze übergeführt, die Salze abgetrennt und die Basen aus den Salzen freigesetzt. Infolge der engen Beziehungen zwischen den neuen Verbindungen in freier Form und in Form ihrer Salze sind im vorausgegangenen und nachfolgend unter freien Verbindungen und Salzen sinn- und zweckgemäss gegebenenfalls auch die entsprechenden Salze bzw. freien Verbindungen zu verstehen.

Ausgangsstoffe und Endprodukte, welche Isomerengemische sind, können nach an sich bekannten Methoden, z.B. durch fraktionierte Destillation, Kristallisation und/oder Chromatographie, in die einzelnen Isomeren getrennt werden. Racemische Produkte können in die optischen Antipoden, z.B. durch Chromatographie und/oder Trennung ihrer diastereomeren Salze, z.B. durch fraktionierte Kristallisation der d- oder ℓ-Camphersulfonate, -Mandelate, -Tartrate oder -Dibenzoyltartrate, getrennt werden.

Die Erfindung betrifft ebenfalls Abänderungen des vorliegenden Verfahrens, wonach ein auf irgendeiner Stufe des Verfahrens erhältliches Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder eines reaktionsfähigen Derivates verwendet wird. Die Erfindung beinhaltet auch daraus resultierende neue Zwischenprodukte.

Beim Verfahren der vorliegenden Erfindung werden vorzugsweise solche Ausgangsstoffe verwendet, welche zu den eingangs als besonders wertvoll geschilderten Verbindungen führen.

Die im Verfahren zur Herstellung der Verbindungen der vorliegenden Erfindung verwendeten Ausgangsstoffe sind bekannt, oder können, falls sie neu sind, nach an sich bekannten Methoden erhalten, z.B. analog wie in den Beispielen beschrieben, hergestellt werden. Neue Ausgangsstoffe bilden ebenfalls einen Gegenstand der Erfindung.

Die bekannten Ausgangsstoffe der Formel II, worin $X_1$ Hydroxy bedeutet, sind in Liebigs Annalen der Chemie, 1976, p. 1663-1673 und in Arch. Pharm. (Weinheim) 310, S. 559-563 (1977) von den oben genannten Autoren beschrieben.

Eine weitere Herstellungsmethode für die Ausgangsstoffe der Formel II besteht in der Oxydation von Verbindungen der allgemeinen Formel V

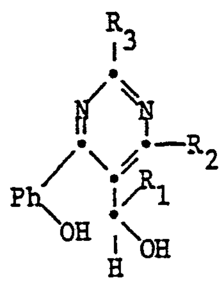

(V),

worin $R_1$ für Wasserstoff steht, gegebenenfalls unter vorübergehenden Schutz der phenolischen Hydroxygruppe.

Als Oxydationsmittel können beispielsweise Dimethylsulfoxyd und Acetanhydrid, N-Chlorsuccinimid und Dimethylsulfid (Corey), Chromsäure und Schwefelsäure in Wasser bei niedrigen Temperaturen, oder die Methode nach Oppenauer (Aluminium-tert.-butoxyd, Aceton und Benzol und solvolytische Abspaltung, z.B. mit verdünnter Schwefelsäure), verwendet werden. Ist die phenolische Hydroxygruppe z.B. durch eine tert.-Butylgruppe geschützt, so wird sie auch in situ freigesetzt.

Ein Ausgangsstoff der Formel II, worin $X_1$ eine metallisierte Hydroxygruppe bedeutet, kann z.B. durch Umsetzung der entsprechenden freien Verbindung ($X_1$ = OH) mit einem Alkalimetall, z.B. Natrium oder Kalium in Gegenwart eines inerten Lösungsmittels, z.B. eines Aethers, wie Diäthyläther, oder eines aromatischen Kohlenwasserstoffs, z.B. Benzol, hergestellt werden.

Ein Ausgangsstoff der Formel II, worin $X_1$ eine veresterte Hydroxygruppe, z.B. ein Halogenatom oder eine Acyloxy- wie Acetyloxy-, Methansulfonyloxy- oder Benzolsulfonyloxygruppe bedeutet, kann ausgehend von der entsprechenden Verbindung, worin $X_1$ für Hydroxy steht, erhalten werden. Die Veresterung kann in an sich bekannter Weise durchgeführt werden.

Die Umwandlung der freien Hydroxygruppe $X_1$ in eine durch eine Halogenwasserstoffsäure veresterte Hydroxygruppe, d.h. in ein Halogenatom, kann üblicherweise durch Behandeln mit einem halogenierenden, insbesondere chlorierenden Mittel vorgenommen werden. Solche Mittel sind z.B. Thionylchlorid, Thionylbromid, Phosphortribromid, Phosphoroxybromid oder -chlorid oder Phosphorpentachlorid, welche man üblicherweise in Gegenwart eines inerten Lösungs- oder Verdünnungsmittels, z.B. Tetrahydrofuran, Dioxan, Methylenchlorid oder Dimethylsulfoxid, einsetzt.

Eine Hydroxygruppe $X_1$ des Ausgangsstoffes der Formel II kann z.B. durch Behandeln mit einem den gewünschten Acylrest einer organischen Carbonsäure einführenden Acylierungsmittel in eine Acyloxygruppe $X_1$ übergeführt werden. Solche Mittel sind z.B. entsprechende Carbonsäuren oder ihre reaktionsfähigen Derivate, wie Anhydride oder Säurehalogenide, z.B. -chloride oder -bromide. Die Umsetzung kann gegebenenfalls in Gegenwart von Kondensationsmitteln, bei Verwendung von freien Carbonsäuren z.B. in Gegenwart von Carbodiimidverbindungen, wie Dicyclohexylcarbodiimid, oder Carbonylverbindungen, wie Diimidazolylcarbonyl, durchgeführt werden. Bei Verwendung von Säurederivaten, z.B. Säurehalogeniden, arbeitet man vorteilhafterweise in Gegenwart eines basischen Mittels, z.B. eines Triniederalkylamins, wie Triäthylamin, oder einer heterocyclischen Base, z.B. Pyridin.

Steht in einem Ausgangsstoff der Formel II der Substituent $X_1$ für Sulfonyloxy, so kann dieser ausgehend von einer entsprechenden Verbindung, worin $X_1$ Hydroxy bedeutet, hergestellt werden. So kann die Hydroxy-Verbindung durch Behandlung mit einem reaktionsfähigen Sulfonsäurederivat, wie einem entsprechenden Halogenid, z.B. Chlorid, wenn notwendig, in Gegenwart eines basischen Mittels für die Neutralisation der entstandenen Säure, in den gewünschten Ausgangsstoff übergeführt werden. Als basische Mittel können die vorher, bei der Herstellung von Carbonsäureestern genannten Verbindungen eingesetzt werden.

Ausgangsstoffe der Formel III, in welchen $X_2$ eine metallisierte, oder eine reaktionsfähige veresterte Hydroxygruppe bedeutet, können analog wie für die Verbindungen der Formel II angegeben, ausgehend von Alkoholen der Formel ROH, hergestellt werden.

Die Ausgangsstoffe der Formel IV des Verfahrens b) können wie folgt hergestellt werden: Die Vorstufe des Ausgangsstoffes, welche in 2-Stellung eine freie Aminogruppe enthält, wird analog zu der in Liebigs Ann. Chem. 1976, S. 1663-1673 beschriebenen Methode, durch Umsetzung von entsprechenden Chromon-2-carbaldehyden mit Guanidin erhalten. Die Umwandlung der Aminogruppe in ein Halogenatom kann beispielsweise durch Ueberführung in ein Diazoniumsalz und Umsetzung mit einem Kupfer-(I)-halogenid vorgenommen werden.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensations- oder Neutralisationsmitteln und/oder in einer inerten Atmosphäre, unter Kühlung, bei Zimmertemperatur oder bei erhöhten Temperaturen, vorzugsweise beim Siedepunkt des verwendeten Lösungsmittels, bei normalem oder erhöhtem Druck durchgeführt.

Von der Erfindung ebenfalls umfasst sind therapeutische Stoffzusammenstellungen, die aus einem lipidsenkend oder analgetisch wirksamen Anteil der Verbindungen der allgemeinen Formel I oder einem Salz und einem pharmakologisch annehmbaren festen Trägerstoff oder flüssigen Verdünnungsmittel bestehen.

Die erfindungsgemässen pharmazeutischen Präparate enthalten mindestens eine Verbindung der allgemeinen Formel I oder ein Salz davon als Wirkstoff zusammen mit einem üblichen pharmazeutischen Trägerstoff. Die Art der Trägerstoffe richtet sich weitgehend nach dem Anwendungsgebiet. Die erfindungsgemässen pharmazeutischen Stoffzusammensetzungen, die als Wirkstoffe Verbindungen der Formel I enthalten, können oral, parenteral oder rektal verabreicht werden.

Zur oralen Behandlung kommen insbesondere feste Doseneinheitsformen, wie Tabletten, Dragées und Kapseln, in Frage, die vorzugsweise zwischen 10 und 90% eines Wirkstoffes der allgemeinen Formel I oder eines Salzes enthalten, um die Verabreichung von täglichen Dosen von 1 bis 50 mg/kg an Warmblütern zu ermöglichen. Zur Herstellung von Tabletten und Dragéekernen kombiniert man die Verbindungen der allgemeinen Formel I mit festen, pulverförmigen Trägerstoffen, wie Lactose, Saccharose, Sorbit, Maisstärke, Kartoffelstärke oder Amylopektin, Cellulosederivaten oder Gelatine, vorzugsweise unter Zusatz von Gleitmitteln, wie Magnesium- oder Calciumstearat, oder Polyäthylenglykolen von geeignetem Molekulargewicht. Dragéekerne überzieht man anschliessend beispielsweise mit konzentrierten Zuckerlösungen, welche z.B. noch arabischen Gummi, Talk und/oder Titandioxid enthalten können, oder mit einem in leicht-flüchtigen organischen Lösungsmitteln oder Lösungsmittelgemischen gelösten Lack. Diesen Ueberzügen können Farbstoffe zugefügt werden, z.B. zur Kennzeichnung verschiedener Wirkstoffdosen. Weiche Gelatinekapseln und andere geschlossene Kapseln bestehen beispielsweise aus einem Gemisch von Gelatine und Glycerin und können z.B. Mischungen einer Verbindung der Formel I mit Polyäthylenglykol enthalten. Steckkapseln enthalten z.B. Granulate eines Wirkstoffes mit festen, pulverförmigen Trägerstoffen, wie z.B. Lactose, Saccharose, Sorbit, Mannit; Stärken, wie Kartoffelstärke, Maisstärke oder Amylopektin, Cellulosederivate und Gelatine sowie Magnesiumstearat oder Stearinsäure.

Als Doseneinheitsformen für die rektale Anwendung kommen z.B. Suppositorien in Betracht, welche aus einer Kombination eines Wirkstoffes mit einer Suppositorien-Grundmasse auf der Basis von natürlichen oder synthetischen Triglyceriden (z.B. Kakaobutter), Polyäthylenglykolen oder geeigneten höheren Fettalkoholen bestehen, und Gelatine-Rektalkapseln, welche eine Kombination des Wirkstoffes mit Polyäthylenglykolen enthalten.

Ampullenlösungen zur parenteralen, insbesondere intramuskulären oder intravenösen Verabreichung enthalten eine Verbindung der Formel I oder ein Salz davon in einer Konzentration von vorzugsweise 0,5 bis 5% als wässerige, mit Hilfe von üblichen Lösungsvermittlern und/oder Emulgiermitteln, sowie gegebenenfalls von Stabilisierungsmitteln bereitete Dispersion, oder vorzugsweise eine wässerige Lösung eines pharmazeutisch annehmbaren, wasserlöslichen Salzes einer Verbindung der allgemeinen Formel I.

Für Flüssigkeiten zur oralen Einnahme, wie Sirups und Elixiere, wird die Konzentration des Wirkstoffes derart gewählt, dass eine Einzeldosis leicht abgemessen werden kann, z.B. als Inhalt eines Teelöffels oder eines Messlöffels von z.B. 5 ml, oder auch als Mehrfaches dieser Volumina.

Die nachfolgenden Beispiele a) bis c) sollen die Herstellung einiger typischer Applikationsformen erläutern, jedoch keineswegs die einzigen Ausführungsformen von solchen darstellen.

a) 250 g Wirkstoff werden mit 550 g Lactose und 292 g Kartoffelstärke vermischt, die Mischung mit einer alkoholischen Lösung von 8 g Gelatine befeuchtet und durch ein Sieb granuliert. Nach dem Trocknen mischt man 60 g Talk, 10 g Magnesiumstearat und 20 g kolloidales Siliciumdioxid zu und presst die Mischung zu 10'000 Tabletten von je 119 mg Gewicht und 25 mg Wirkstoffgehalt, die gewünschtenfalls mit Teilkerben zur feineren Anpassung der Dosierung versehen sein können.

b) Aus 100 g Wirkstoff, 379 g Lactose und der alkoholischen Lösung von 6 g Gelatine stellt man ein Granulat her, das man nach dem Trocknen mit 10 g kolloidalem Siliciumdioxid, 40 g Talk, 60 g Kartoffelstärke und 5 g Magnesiumstearat mischt und zu 10'000 Dragéekernen presst. Diese werden anschliessend mit einem konzentrierten Sirup aus 533,5 g krist. Saccharose, 20 g Schellack, 75 g arabischem Gummi, 250 g Talk, 20 g kolloidalem Siliciumdioxid und 1,5 g Farbstoff überzogen und getrocknet. Die erhaltenen Dragées wiegen je 150 mg und enthalten je 10 mg Wirkstoff.

c) 25 g Wirkstoff und 1975 g fein geriebene Suppositoriengrundmasse (z.B. Kakaobutter) werden gründlich gemischt und dann geschmolzen. Aus der durch Rühren homogen gehaltenen Schmelze werden 1000 Suppositorien von 2 g gegossen. Sie enthalten je 25 mg Wirkstoff.

Die folgenden Beispiele dienen zur Illustration der Erfindung, sollten jedoch den Umfang derselben in keiner Weise beschränken. Temperaturen werden in Celsiusgraden angegeben, und die Angaben über Teile betreffen Gewichtsteile. Wenn nicht anders definiert, wird das Eindampfen von Lösungsmitteln unter vermindertem Druck, z.B. zwischen ungefähr 0,1 und 15 mmHg, durchgeführt.

Beispiel 1: Eine Lösung von 8 g 2-Phenyl-5-hydroxy-5H-[1]benzopyrano-[4,3-d]pyrimidin und 5 ml 10-normaler äthanolischer Salzsäure in 200 ml Aethanol wird 2 Stunden unter Wasserausschluss am Rückfluss gekocht.

Das Reaktionsgemisch wird dann unter vermindertem Druck zur Trockene eingedampft, mit Eis und 20 ml gesättigter wässeriger Natriumcarbonatlösung versetzt und mit Diäthyläther dreimal extrahiert. Die Aether-Extrakte werden über Natriumsulfat getrocknet und unter vermindertem Druck eingedampft. Der Rückstand wird aus Pentan umkristallisiert. Man erhält das 2-Phenyl-5-äthoxy-5H-[1]benzopyrano[4,3-d]pyrimidin der Formel

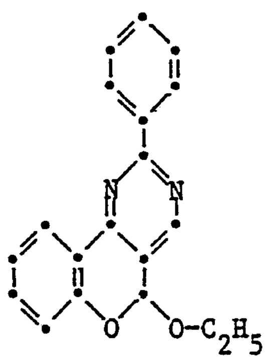
,

welches bei 79-80° schmilzt.

Beispiel 2: Zu einer Lösung von 8 g 2-Phenyl-5-hydroxy-5H-[1]benzopyrano[4,3-d]pyrimidin in 300 ml Methanol fügt man 3,3 g konz. Schwefelsäure und kocht 5 1/2 Stunden am Rückfluss unter Wasserausschluss. Dann stellt man das Reaktionsgemisch mit 0,5-normaler wässeriger Natriumhydroxydlösung unter Eiskühlung auf den pH-Wert 8 und extrahiert mit Essigsäureäthylester und Diäthyläther. Die organischen Extrakte werden über Natriumsulfat getrocknet und unter vermindertem Druck eingedampft. Der Rückstand wird aus Benzol-Diäthyläther umkristallisiert. Man erhält das 2-Phenyl-5-methoxy-5H-[1]benzopyrano[4,3-d]pyrimidin, welches bei 142-143° schmilzt.

Beispiel 3: Analog zu dem im Beispiel 2 beschriebenen Verfahren erhält man ausgehend von 2-Phenyl-5-hydroxy-5H-[1]benzopyrano[4,3-d]-pyrimidin und dem entsprechenden Alkanol [1) Propanol, 2) Butanol, 3) Isopropanol, 4) Pentanol, 5) Hexanol] die folgenden 2-Phenyl-5-alkyloxy-5H-[1]benzopyrano[4,3-d]pyrimidin-Verbindungen:

1) 2-Phenyl-5-propyloxy-5H-[1]benzopyrano[4,3-d]pyrimidin. Extrahiert mit Dichlormethan. F. 68-69° nach Umkristallisation aus Propanol.

2) 2-Phenyl-5-butyloxy-5H-[1]benzopyrano[4,3-d]pyrimidin. Extrahiert mit Dichlormethan. F. 66-69° (aus Butanol).

3) 2-Phenyl-5-isopropyloxy-5H-[1]benzopyrano[4,3-d]pyrimidin. Extrahiert mit Dichlormethan. F. 90-92° (aus Isopropanol).

4) 2-Phenyl-5-pentyloxy-5H-[1]benzopyrano[4,3-d]pyrimidin. Extrahiert mit Dichlormethan, F. 69-70° (aus Pentan).

5) 2-Phenyl-5-hexyloxy-5H-[1]benzopyrano[4,3-d]pyrimidin. Extrahiert mit Dichlormethan. F. 69-70° (aus Pentan).

Beispiel 4: Ausgehend von 10 g 8,9-Trimethylen-2-phenyl-5-hydroxy-5H[1]benzopyrano[4,3-d]pyrimidin erhält man analog zu dem im Beispiel 2 beschriebenen Verfahren das 8,9-Trimethylen-2-phenyl-5-äthoxy-5H-[1]benzopyrano[4,3-d]pyrimidin, welches nach Umkristallisation aus Diäthyläther bei 135-136° schmilzt.

Der Ausgangsstoff wird ausgehend von 6,7-Trimethylen-4H-4-oxo-[1]benzopyran-3-carboxaldehyd [beschrieben in DOS 751 211; Pat. Nr. 2,523,194; Haas et al., Ciba-Geigy AG] und Benzamidin, nach dem in Ann. Chem., 1976, 1663 beschriebenen Verfahren hergestellt. Das 8,9-Trimethylen-2-phenyl-5-hydroxy-5H-[1]benzopyrano[4,3-d]pyrimidin schmilzt bei 213-214° (umkristallisiert aus Aethanol).

Beispiel 5: In Analogie zu dem im Beispiel 4 beschriebenen Verfahren erhält man ausgehend von den entsprechenden Hydroxyverbindungen und Aethanol die folgenden Verbindungen:

a) 8,9-Tetramethylen-2-phenyl-5-äthoxy-5H-[1]benzopyrano[4,3-d]pyrimidin, welches nach Umkristallisation aus Diäthyläther und dann aus Benzin, bei 142-145° schmilzt,

b) 8-Phenyl-11-äthoxy-11H-naphtho[1',2'; 7,8]pyrano[4,3-d]pyrimidin, welches nach Umkristallisation aus Essigsäureäthylester-Diäthyläther bei 151-153° schmilzt.

Die Ausgangsmaterialien lassen sich leicht ausgehend von den in DOS 2,523,194 (vgl. Beispiel 4) beschriebenen Aldehyden herstellen:

Ausgangsstoff für a): 8,9 Tetramethylen-2-phenyl-5-hydroxy-5H-[1]benzopyrano[4,3-d]pyrimidin, F. 213-214° (aus Aethanol).

Ausgangsstoff für b): 8-Phenyl-11-hydroxy-11H-naphtho[1',2'; 7,8]pyrano[4,3-d]pyrimidin, F. 224-227° (aus Aethanol).

Beispiel 6: Analog zu dem in Beispiel 2 beschriebenen Verfahren erhält man ausgehend von den in 2-Stellung entsprechend substituierten 2-$R_3$-5-hydroxy-5H-[1]benzopyrano[4,3-d]pyrimidinen die folgenden 2-$R_3$-5-OR-5H-[1]benzopyrano[4,3-d]pyrimidine der Formel I, in welchen der Phenylenrest Ph unsubstituiert ist, $R_1$ und $R_2$ Wasserstoff bedeuten und die weiteren Symbole die folgenden Bedeutungen haben:

| Verbindung | $R_3$ | R | Extrahiert mit | Umkristallisiert aus | F. |
|---|---|---|---|---|---|
| 1 | 2-Pyridyl | Aethyl | Dichlormethan | Diäthyläther | 140-141° |
| 2 | 4-Pyridyl | Propyl | Dichlormethan | Diäthyläther | 115-116° |
| 3 | 3-Pyridyl | Aethyl | Diäthyläther-Essigsäureäthylester | Diäthyläther-Essigsäureäthylester | 98-100° |
| 4 | 3-Methoxy-phenyl | Propyl | Dichlormethan | Diäthyläther-Pentan | 72-73° |
| 5 | 4-Cyclohexyl-phenyl | Propyl | - | Propanol | 99-100° |
| 6 | 4-Chlor-phenyl | Propyl | - | Propanol | 101° |
| 7 | 3-Methyl-phenyl | Propyl | Dichlormethan | Pentan | 86° |
| 8 | 4-Aethoxycarbonylmethyl-phenyl | Aethyl | Diäthyläther-Essigsäureäthylester | Dichlormethan Diäthyläther | 115° |

Die als Ausgangsstoffe verwendeten 2-$R_3$-5-Hydroxy-5H-[1]benzopyrano[4,3-d]pyrimidine werden analog zu dem in Ann. Chem., 1976, 1663 beschriebenen Verfahren ausgehend von 4-Oxo-4H-[1]benzopyran-3-carboxaldehyd und dem entsprechenden Amidin der Formel $R_3$-CNHNH$_2$ hergestellt:

| Ausgangsstoff: $R_3$: | | Umkristallisation aus: | F: |
|---|---|---|---|
| 1 | 2-Pyridyl | Aethanol-Wasser | 220° |
| 2 | 4-Pyridyl | Aethanol-Wasser | 275-280° |
| 3 | 3-Pyridyl | Aethanol-Wasser | 270-275° |
| 4 | 3-Methoxy-phenyl | Aethanol-Wasser | 168-169,5° |
| 5 | 4-Cyclohexyl-phenyl | Aethanol-Wasser | 188-190° |
| 6 | 4-Chlor-phenyl | Aethanol-Wasser | 208-210° |
| 7 | 3-Methyl-phenyl | Aethanol-Wasser | 167-168° |
| 8 | 4-Aethoxycarbonylmethyl-phenyl | Aethanol-Wasser | 112-115° |

Beispiel 7: Eine Lösung von 8 g 2-(4-Pyridyl)-5-hydroxy-5H-[1]benzopyrano[4,3-d]pyrimidin in 200 ml n-Propanol wird mit 6 g p-Toluolsulfonsäure versetzt und 3 Stunden am Rückfluss gekocht. Dann dampft man das Reaktionsgemisch unter vermindertem Druck auf ein Volumen von ungefähr 50 ml ein und erhält so das kristalline 2-(4-Pyridyl)-5-propyloxy-5H-[1]benzopyrano[4,3-d]pyrimidin-tosylat, welches bei 188-190° schmilzt.

Beispiel 8: In Analogie zu dem im Beispiel 2 beschriebenen Verfahren erhält man ausgehend von entsprechenden 5-Hydroxyverbindungen und Propanol bzw. Isopropanol die folgenden Verbindungen:

1) 8,9-Dimethyl-2-phenyl-5-propyloxy-5H-[1]benzopyrano[4,3-d]pyrimidin, welches nach Umkristallisation aus Propanol bei 167-168° schmilzt.

2) 9-Aethyl-2-phenyl-5-propyloxy-5H-[1]benzopyrano[4,3-d]pyrimidin, F. 82-83° (aus Pentan).

3) 7-Methyl-2-phenyl-5-propyloxy-5H-[1]benzopyrano[4,3-d]pyrimidin, F. 100-101° (aus Propanol).

4) 9-Chlor-2-phenyl-5-propoxy-5H-[1]benzopyrano[4,3-d]pyrimidin, F. 122-123° (aus Propanol).

5) 9-Chlor-2-phenyl-5-isopropyloxy-5H-[1]benzopyrano[4,3-d]pyrimidin, F. 149-150° (aus Pentan).

Die als Ausgangsstoffe verwendeten entsprechenden 5-Hydroxy-Verbindungen weisen die folgenden Schmelzpunkte auf

| Ausgangsstoff: | Umkristallisiert aus: | F.: |
|---|---|---|
| 1) | Aethanol/Wasser | 220-222° |
| 2) | Aethanol/Wasser | 178-179° |
| 3) | Aethanol/Wasser | 192-194° |
| 4) und 5) | Aethanol/Wasser | 199-201°. |

Beispiel 9: 117 g 2-(4-Methoxy-phenyl)-5-hydroxy-5H-[1]benzopyrano[4,3-d]pyrimidin werden mit 800 ml n-Propanol und 0,2 ml konz. Schwefelsäure 2 Stunden unter Wasserausschluss am Rückfluss gekocht. Dann wird die Lösung bis zur Hälfte eingedampft und das Produkt im Kühlschrank bei 0° zum Kristallisieren gebracht. Nach dem Filtrieren und Waschen mit kaltem Petroläther erhält man das 2-(4-Methoxy-phenyl)-5-propyloxy-5H-[1]benzopyrano[4,3-d]pyrimidin, welches bei 104-105° schmilzt.

Beispiel 10: Analog zu dem in Beispiel 9 beschriebenen Verfahren erhält man ausgehend von der entsprechenden 5-Hydroxy-Verbindung und n-Propanol bzw. n-Butanol die folgenden Verbindungen:

1) 2-Methyl-5-propyloxy-5H-[1]benzopyrano[4,3-d]pyrimidin, F. 48-49° (aus Pentan bei -60°).

2) 2-(3,4-Dimethoxy-benzyl)-5-propyloxy-5H-[1]benzopyrano[4,3-d]-pyrimidin. F. 73-75° (aus Propanol).

3) 2-(3,5-Dimethoxy-phenyl)-5-propyloxy-5H-[1]benzopyrano[4,3-d]-pyrimidin. F. 85-86° (aus Petroläther).

4) 2-(3-Pyridyl)-5-butyloxy-5H-[1]benzopyrano[4,3-d]pyrimidin. F. 61-62° (aus Petroläther).

5) 2-(4-Hydroxy-phenyl)-5-butyloxy-5H-[1]benzopyrano[4,3-d]pyrimidin. F. 220°unter Zersetzung (aus Essigsäureäthylester).

6) 2-Phenyl-5-butyloxy-9-chlor-5H-[1]benzopyrano[4,3-d]pyrimidin. F. 120-122° (aus Methylenchlorid-Dimethyläther).

7) 2-(5-Methyl-phenyl)-5-butyloxy-5H-[1]benzopyrano[4,3-d]pyrimidin. F. 76-78° (aus Butanol).

8) 2-(4-Methoxy-phenyl)-5-butyloxy-5H-[1]benzopyrano[4,3-d]pyrimidin. F. 82-84° (aus Butanol).

9) 2-(3-Methoxy-phenyl)-5-butyloxy-5H-[1]benzopyrano[4,3-d]pyrimidin. F. 60° (aus Pentan).

10) 2-(4-Aethoxycarbonyl-phenyl)-5-butyloxy-5H-[1]benzopyrano[4,3-d]-pyrimidin. F. 91-92° (aus Petroläther).

11) 2-(4-Aethoxycarbonylmethyl-phenyl)-5-butyloxy-5H-[1]benzopyrano-[4,3-d]pyrimidin. F. 80-82° (aus Petroläther).

Der für die obige Verbindung 4) verwendete Ausgangsstoff, nämlich das 2-(3-Pyridyl)-5-hydroxy-5H-[1]benzopyrano[4,3-d]pyrimidin wird ausgehend von 4-Oxo-[1]benzopyrano-3-carboxaldehyd und 3-Pyridylamidin, gemäss dem in Ann. Chem., 1976, 1663 beschriebenen Verfahren hergestellt. F. 230° unter Zersetzung (aus Aethanol).

Beispiel 11: Analog zu dem im Beispiel 1 beschriebenen Verfahren erhält man ausgehend von 2-Phenyl-5-methyl-5-hydroxy-[1]benzopyrano-[4,3-d]pyrimidin und Aethanol das 2-Phenyl-5-methyl-5-äthoxy-[1]-benzopyrano[4,3-d]pyrimidin, dessen Hydrochlorid bei 180° schmilzt.

Der Ausgangsstoff kann ausgehend von 3-Acetyl-chromon und Benzamidin nach dem in Ann. Chem. 1976, 1663 beschriebenen Verfahren hergestellt werden. Die 5-Hydroxy-Verbindung schmilzt bei 240-245° nach Umkristallisation aus Aethanol.

Beispiel 12: Analog zu dem im Beispiel 1 beschriebenen Verfahren erhält man auch die folgenden Verbindungen:

1) 2-(4-Hydroxy-phenyl-)-5-äthyloxy-5H-[1]benzopyrano[4,3-d]pyrimidin. F. 210° (aus Essigsäureäthylester).

2) 2-(4-Methoxycarbonyl-phenyl)-5-äthyloxy-5H-[1]benzopyrano[4,3-d]-pyrimidin. F. 161-163° (aus Aethanol).

Beispiel 13: In Analogie zu dem im Beispiel 7 beschriebenen Verfahren erhält man ausgehend von den entsprechenden 5-Hydroxy-Verbindungen und dem jeweiligen Alkanol die folgenden Verbindungen:

1) 2-(3-Pyridyl)-5-methoxy-5H-[1]benzopyrano[4,3-d]pyrimidin, F. 197-199° (umkristallisiert aus Methylenchlorid-Diäthyhläther).

2) 2-(3-Pyridyl)-5-propyloxy-5H-[1]benzopyrano[4,3-d]pyrimidin, F.102-103° (aus Methylenchlorid-Diäthyläther).

3) 8-Aethyl-2-(3-pyridyl)-5-butyloxy-5H-[1]benzopyrano[4,3-d]pyrimidin, F. 75-76° (aus Methylenchlorid-Diäthyläther).

4) 6-Methyl-2-(3-pyridyl)-5-butyloxy-5H-[1]benzopyrano[4,3-d]pyrimidin, F. 99-100° (aus Diäthyläther-Petroläther).

5) 2-(4-Pyridyl)-5-methoxy-5H-[1]benzopyrano[4,3-d]pyrimidin, F. 179-180° (aus Methylenchlorid-Diäthyläther).

6) 2-(4-Pyridyl)-5-butyloxy-5H-[1]benzopyrano[4,3-d]pyrimidin, F. 102-103° (aus Diäthyläther-Petroläther).

Die als Ausgangsstoffe verwendeten, in den vorhergehenden Beispielen noch nicht enthaltenen 2-$R_3$-5-Hydroxy-5H-[1]benzopyrano-[4,3-d]pyrimidine erhält man analog zu dem in Ann. Chem., 1976, 1663 beschriebenen Verfahren, ausgehend von entsprechend substituierten

4-Oxo-4H-[1]benzopyran-3-carboxaldehyden und dem entsprechenden Amidin $R_3$-$CNHNH_2$:

8-Aethyl-2-(3-pyridyl)-5-hydroxy-5H-[1]benzopyrano[4,3-d]pyrimidin, F. 195° (aus Aethanol).

6-Methyl-2-(3-pyridyl)-5-hydroxy-5H-[1]benzopyrano[4,3-d]pyrimidin, F. 245-247° (aus Aethanol).

Beispiel 14: Zu einer Lösung von 5 g 2-(4-Methoxy-phenyl)-5-propyloxy-5H-[1]benzopyrano[4,3-d]pyrimidin in 50 ml Isopropanol fügt man 20 ml von, mit Chlorwasserstoff gesättigtem Isopropanol hinzu und lässt das Gemisch über Nacht bei Zimmertemperatur stehen. Das Kristallisat wird abgenutscht, mit Aether gewaschen und getrocknet. Man erhält das 2-(4-Methoxy-phenyl)-5-isopropyloxy-5H-[1]benzopyrano-[4,3-d]pyrimidin-hydrochlorid, welches bei 168-170° schmilzt.

Beispiel 15: Eine Lösung von 5 g 2-(4-Methoxyphenyl)-5-propionyloxy-5H-[1]benzopyrano[4,3-d]pyrimidin in 100 ml Aethanol wird eine Stunde unter Rückfluss gekocht. Das Reaktionsgemisch wird dann bis zur Trübung mit Wasser versetzt und über Nacht auf Zimmertemperatur abkühlen gelassen. Das Kristallisat wird abgenutscht, mit wenig Aethanol und Aether gewaschen und getrocknet. Man erhält das 2-(4-Methoxy-phenyl)-5-äthoxy-5H-[1]benzopyrano[4,3-d]pyrimidin, welches bei 106-108° schmilzt.

Das Ausgangsmaterial wird wie folgt hergestellt: Zu einer Lösung von 5 g 2-(4-Methoxy-phenyl)-5-hydroxy-5H-[1]benzopyrano[4,3-d] pyrimidin in 50 ml wasserfreiem Tetrahydrofuran und 2,4 ml Triäthylamin fügt man unter Rühren in wasserfreier Atmosphäre bei -5° tropfenweise 1,45 ml Propionylchlorid hinzu. Das Reaktionsgemisch wird anschliessend 2 Stunden bei Zimmertemperatur gerührt, dann auf Wasser gegossen und mit Aether extrahiert. Die vereinigten organischen Phasen werden zweimal mit Wasser gewaschen, über Natriumsulfat getrocknet und unter vermindertem Druck bei 22° eingedampft. Der Rückstand wird aus Methylenchlorid-Petroläther umkristallisiert. Man erhält das 2-(4-Methoxy-phenyl)-5-propionyloxy-5H-[1]benzopyrano[4,3-d]-pyrimidin, welches bei 130-131° schmilzt.

Beispiel 16: Analog zu dem im Beispiel 2 beschriebenen Verfahren erhält man ausgehend von 2-(4-Chlormethyl-phenyl)-5-hydroxy-5H-[1]-benzopyrano[4,3-d]pyrimidin und Propanol:

1) das 2-(4-Chlormethyl-phenyl)-5-propyloxy-5H-[1]benzopyrano[4,3-d] pyrimidin,. welches nach Umkristallisation aus n-Propanol bei 103-104° schmilzt; und ausgehend von 2-(4-Hydroxymethyl-phenyl)-5-hydroxy-5H-[1]benzopyrano[4,3-d]pyrimidin und Butanol:

2) das 2-(4-Hydroxymethyl-phenyl)-5-propyloxy-5H-[1]benzopyrano[4,3-d] pyrimidin, F. 122-123° (aus n-Propanol).

Die Ausgangsstoffe werden analog zum Beispiel 4, ausgehend von 4H-4-Oxo-[1]benzopyran-3-carboxaldehyd und dem Benzamidin, welches in 4-Stellung durch Chlormethyl bzw. Hydroxymethyl substituiert ist, hergestellt. Man erhält

1a) das 2-(4-Chlormethyl-phenyl)-5-hydroxy-5H-[1]benzopyrano[4,3-d]-pyrimidin, F. 169-170° (aus Aethanol) und

2 a) das 2-(4-Hydroxymethyl-phenyl)-5-hydroxy-5H-[1]benzopyrano-[4,3-d]pyrimidin, F. 200-201° (aus Aethanol).

Beispiel 17: Eine Lösung von 1,5 g 2-(4-Methoxy-phenyl)-5-hydroxy-5H-[1]benzopyrano[4,3-d]pyrimidin in 50 ml Benzol wird mit 0,57 g Orthoameisensäuretriäthylester un 1 Tropfen konz. Schwefelsäure versetzt und 2 Stunden unter Rückfluss gekocht. Dann dampft man zur Trockene ein, verteilt den Rückstand zwischen Methylenchlorid und gesättigter wässeriger Natriumbicarbonatlösung, wäscht die organische Phase zweimal mit Wasser, trocknet über Natriumsulfat und

dampft unter vermindertem Druck zur Trockene ein. Der Rückstand wird aus Methylenchlorid-Aether umkristallisiert. Man erhält das 2-(4-Methoxy-phenyl)-5-äthoxy-5H-[1]benzopyrano[4,3-d]pyrimidin, welches bei 105-107° schmilzt.

Beispiel 18: Zu einer Suspension von 361 mg Magnesium in wenig abs. Tetrahydrofuran fügt man in einer wasserfreien Atmosphäre unter Rühren tropfenweise 2,25 g 4-Brom-anisol in 10 ml abs. Tetrahydrofuran hinzu. Nach beendeter Zugabe wird 90 Minuten bei 60-70° weitergerührt. Dann kühlt man auf Zimmertemperatur ab und versetzt das Reagens mit einer Lösung von 3,20 g 2-Methylsulfonyl-5-propyloxy-5H-[1]benzopyrano]4,3-d]pyrimidin in 20 ml abs. Tetrahydrofuran und lässt anschliessend 16 Stunden bei Zimmertemperatur weiterrühren. Die nun dunkelbraune Reaktionslösung wird unter vermindertem Druck zur Trockene eingedampft. Den Rückstand verteilt man zwischen dreimal 100 ml Chloroform und 100 ml gesättigter wässeriger Ammoniumchloridlösung. Die organischen Phasen werden neutralgewaschen, über wasserfreiem Natriumsulfat getrocknet und unter vermindertem Druck zur Trockene eingedampft. Chromatographische Reinigung des so erhaltenen braunen Oels an 120 g Silicagel mit Chloroform als Eluens liefert das reine 2-(4-Methoxy-phenyl)-5-propyloxy-5H-[1]benzopyrano[4,3-d]-pyrimidin. F. 102-104° (aus n-Propanol-Petroläther).

Der Ausgangsstoff wird wie folgt hergestellt: Zu 37 g 2-Methylthio-5-hydroxy-5H-[1]benzopyrano[4,3-d]pyrimidin in 250 ml n-Propanol fügt man unter Rühren bei 100° 1 ml konz. Schwefelsäure und kocht anschliessend 2 Stunden unter Rückfluss. Dann wird auf ein Volumen von ungefähr 50 ml eingedampft, mit Eis auf 0° gekühlt und zwischen eiskalter wässeriger 2-normaler Natriumcarbonatlösung und Methylenchlorid verteilt. Die organischen Phasen werden mit Wasser gewaschen, über wasserfreiem Natriumsulfat getrocknet und unter vermindertem Druck bis zur einsetzenden Kristallisation eingedampft. Man erhält das 2-Methylthio-5-propyloxy-5H-[1]benzopyrano[4,3-d]pyrimidin, welches bei 112-114° schmilzt.

Zu 7,2 g der obigen Verbindung in 100 ml abs. Chloroform fügt man bei Zimmertemperatur portionenweise 10,35 g m-Chlor-perbenzoesäure (90 %ig) hinzu und rührt über Nacht bei Zimmertemperatur unter Wasserausschluss. Dann verteilt man die Reaktionsmischung zwischen 200 ml Chloroform und 2-mal 200 ml gesättigter wässeriger Natriumbisulfitlösung. Die organischen Extrakte werden sukzessive mit 2-mal 200 ml wässeriger Natriumbicarbonatlösung und Wasser gewaschen, über wasserfreiem Natriumsulfat getrocknet und unter vermindertem Druck eingedampft. Aus dem Eindampfrückstand erhält man mit Aether das 2-Methylsulfonyl-5-propyloxy-5H-[1]benzopyrano[4,3-d]pyrimidin. F. 141-143°.

Beispiel 19: Zu einer Suspension von 160 mg Natriumhydrid (50 %ig in Mineralöl) in 15 ml abs. Dimethylformamid fügt man 1 g 2-(4-Methoxyphenyl)-5-hydroxy-5H-[1]benzopyrano[4,3-d]pyrimidin und lässt bei Zimmertemperatur bis zum Abschluss der Wasserstoffentwicklung rühren. Dann fügt man langsam 600 mg Propyljodid hinzu und rührt zwei Stunden bei 40-50° weiter. Dann giesst man auf 200 ml Eiswasser und extrahiert mit 3-mal 50 ml Methylenchlorid. Die organischen Phasen werden mit Wasser gewaschen, über Natriumsulfat getrocknet und unter vermindertem Druck eingedampft. Nach Abdestillieren des überschüssigen Dimethylformamids im Hochvakuum erhält man aus dem Eindampfrückstand nach Umkristallisation aus n-Propanol-Aether das 2-(4-Methoxy-phenyl)-5-propyloxy-5H-[1]benzopyrano]4,3-d]pyrimidin, welches bei 102-103° schmilzt.

Beispiel 20: Auf analoge Weise wie im Beispiel 19 beschrieben erhält man ausgehend von 2-(4-Hydroxy-phenyl)-5-butyloxy-5H-[1]benzopyrano-[4,3-d]pyrimidin nach Behandlung mit Natriumhydrid und Methyljodid das 2-(4-Methoxy-phenyl)-5-butyloxy-5H-[1]benzopyrano[4,3-d]pyrimidin, welches bei 82-84° schmilzt.

Beispiel 21: Zu 350 mg 2-(4-Hydroxy-phenyl)-5-butyloxy-5H-[1]benzopyrano[4,3-d]pyrimidin in 15 ml abs.n-Butanol fügt man eine Lösung von 32 mg Natrium in 5 ml abs. n-Butanol hinzu. Die nun gelbe Lösung

wird zur Trockene eingedampft. Man erhält das Natriumsalz der oben genannten Verbindung als gelbes Pulver, welches über 300° schmilzt.

Beispiel 22: Eine Lösung 300 mg Natrium in 5 ml abs. Methanol wird unter vermindertem Druck zur Trockene eingedampft. Den Rückstand versetzt man unter wasserfreien Bedingungen mit einer Lösung von 1g 2-[4-Nitrophenyl)-5-propyloxy-5H-[1]benzopyrano[4,3-d]pyrimidin in 5 ml abs. N,N-Dimethylformamid und lässt 24 Stunden bei Zimmertemperatur stehen. Dann versetzt man mit 100 ml Wasser und extrahiert das Reaktionsgemisch mit 3-mal 50 ml Aether. Die organischen Phasen werden neutralgewaschen, über Natriumsulfat getrocknet und unter vermindertem Druck zur Trockene eingedampft. Chromatographische Reinigung des Eindampfrückstandes an Kieselgel mit Chloroform als Eluens sowie anschliessende Kristallisation aus Aether liefert das 2-(4-Methoxy-phenyl)-5-propyloxy-5H-[1]benzopyrano[4,3-d]pyrimidin. F. 97-100°.

Das Ausgangsmaterial erhält man ausgehend von p-Nitrobenzamidin und 3-Formylchromon, wobei man als Zwischenprodukt das 2-(4-Nitrophenyl)-5-hydroxy-5H-[1]benzopyrano]4,3-d]pyrimidin(F = 220-222°) erhält. Die Behandlung dieser Verbindung mit Schwefelsäure und n-Propanol ergibt das 2-(4-Nitrophenyl)-5-propyloxy-5H-[1]benzopyrano-[4,3-d]pyrimidin, welches bei 160-162° schmilzt. (Diese Verbindung ist auch ein Endprodukt der Erfindung).

Beispiel 23: Analog zum im Beispiel 2 beschriebenen Verfahren erhält man ausgehend von 2-(3-Trifluromethylphenyl)-5-hydroxy-5H-[1]benzopyrano[4,3-d]pyrimidin (F. 205-207°; aus Aethanol/Wasser) und n-Propanol das 2-(3-Trifluormethyl-phenyl)-5-propyloxy-5H-[1]benzopyrano-[4,3-d]pyrimidin, welches nach Umkristallisation aus n-Propanol bei 117-118° schmilzt.

Beispiel 24: Zu einer warmen Lösung von 5 g 2-(4-Methoxy-phenyl)-5-propyloxy-5H-[1]benzopyrano[4,3-d]pyrimidin in 60 ml n-Propanol fügt

man 0,72 g konz. Schwefelsäure hinzu. Dann kühlt man auf Zimmertemperatur, versetzt mit 50 ml Aether und filtriert ab. Man erhält das 2-(4-Methoxy-phenyl)-5-propyloxy-5H-[1]benzopyrano[4,3-d]pyrimidin-hemisulfat, welches bei 185° schmilzt.

Bei Verwendung von Methansulfonsäure erhält man analog das entsprechende Methansulfonat, welches bei 182-184° schmilzt.

Mit Chlorwasserstoff wird das entsprechende Hydrochlorid erhalten. F. 180°.

Beispiel 25: Ein Gemisch von 5 g 2-(4-Nitro-phenyl)-5-propyloxy-5H-[1]benzopyranol[4,3-d]pyrimidin, 50 ml Dioxan und 1 g Raney-Nickel wird bei Zimmertemperatur und Normaldruck bis zum Verbrauch von 3 Aequivalenten Wasserstoff hydriert. Man filtriert den Katalysator ab, dampft die Lösung ein und kristallisiert den Rückstand aus Aether-Petroläther um. Man erhält das 2-(4-Amino-phenyl)-5-propyloxy-5H-[1]benzopyrano[4,3-d]pyrimidin, welches bei 125-127° schmilzt.

Methodik zur Prüfung auf Wirkung auf Lipide und Lipoproteine im Serum

Männliche Ratten von 180-200 g Körpergewicht erhalten an 3 Tagen einmal täglich peroral die Prüfsubstanz gelöst in Polyäthylenglykol 400. Am 4. Tag werden die Tiere zweimal, morgens und abends, behandelt. Nach der letzten Behandlung wird den Tieren das Futter entzogen. Sechzehn Stunden später werden die Ratten getötet. Aus dem Blut wird Serum gewonnen.

Gleiche Mengen Serum von je 2 Ratten aus derselben Gruppe werden vereinigt, und 0.05 EDTA (Aethylendiamintetraessigsäure) und 0,01 % Thimerosal werden zugesetzt. Die Serumlipoproteine werden durch Ultrazentrifugieren in einem Beckman 40.3 Rotor isoliert. Die VLDL (very low density lipoproteins) werden durch 24-stündiges

Zentrifugieren bei 78 000 g und der Dichte 1.006 gewonnen, die LDL (low density lipoproteins) durch 24-stündiges Zentrifugieren bei 78 000 g und der Dichte 1,040, und die HDL (high density lipoproteins) durch 24-stündiges Zentrifugieren bei 109 000 g und der Dichte 1.21.

In jeder Serumprobe und in jeder Lipoproteinfraktion werden Cholesterin und Triglyceride mit enzymatischen Methoden bestimmt. LDL-Cholesterin wurde als wichtigster Parameter für die Beurteilung der Lipidsenkung betrachtet.

Literatur: K.R. Müller und R.G. Cortesi, Artery 4(6):564-577 (1978).

Die Zahlen der folgenden Resultate geben die Aenderungen vom LDL-Cholesterin in % der Kontrolle an. Dosierung: 50 mg/kg peroral.

2-Phenyl-5-äthoxy-5H-[1]benzopyrano[4,3-d]pyrimidin: -61 %.
2-(3-Methoxy-phenyl)-5-propyloxy-5H-[1]benzopyrano[4,3-d]pyrimidin: -43 %.
2-(4-Methoxy-phenyl)-5-propyloxy-5H-[1]benzopyrano[4,3-d]pyrimidin: -38 %.
2-(5-Methyl-phenyl)-5-butyloxy-5H-[1]benzopyrano[4,3-d]pyrimidin: -29 %.
2-(4-Hydroxy-phenyl)-5-äthyloxy-5H-[1]benzopyrano[4,3-d]pyrimidin: -69 %.

Methodik zur Prüfung der analgetischen Wirkung

Diese Wirkung wird durch Auswertung des Krümmungssyndroms an der Ratte bestimmt. Das Krümmungssyndrom wird z.B. in weiblichen Ratten von 120-140 g (5 Tiere pro Gruppe) durch eine intraperitoneale Injektion von 0,2 ml einer 3 %igen Essigsäurelösung eine Stunde nach der oralen Verabreichung der geprüften Substanz, hervorgerufen. Die Krümmungs-Bewegungen der Versuchstiere werden während 20 Minuten gezählt.

Die analgetische (antinociceptive) Wirksamkeit einer Verbindung wird durch die Bestimmung der Dosis (durch Interpolieren), welche bei Kontrolltieren (die lediglich den Trägerstoff der Wirksubstanz erhalten haben) die Anzahl der Krümmungen um 50 % vermindert, beurteilt. (= $ED_{50}$).

Literatur: Ziel et al., Pain Res. and Therapy, Vol. 1,ed. Bonica and Albe, Raven Press N.Y., 1976, p. 517.

Die $ED_{50}$-Werte sind im folgenden in mg/kg bei peroraler Verabreichung zu verstehen:

2-(4-Pyridyl)-5-propyloxy-5H-[1]benzopyrano[4,3-d]pyrimidin: 15,0 mg/kg,

2-(3-Pyridyl)-5-äthoxy-5H-[1]benzopyrano[4,3-d]pyrimidin: 1,5 mg/kg.

2-(3-Pyridyl)-5-butyloxy-5H-[1]benzopyrano[4,3-d]pyrimidin: 5mg/kg.

6-Methyl-2-(3-pyridyl)-5-butyloxy-5H-[1]benzopyrano[4,3-d]pyrimidin: 60 mg/kg.

1-(2-Pyridyl)-5-äthoxy-5H-[1]benzopyrano[4,3-d]pyrimidin: 15 mg/kg.

Patentansprüche
(für alle benannten Länder ausser Oesterreich)

1. Pharmazeutische Präparate enthaltend substituierte Aether von 5-Hydroxy-5H-[1]benzopyrano[4,3-d]pyrimidinen der allgemeinen Formel I

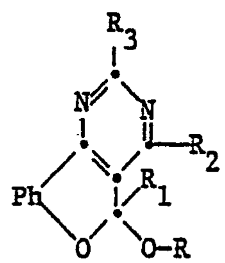

(I) ,

worin Ph einen gegebenenfalls substituierten 1,2-Phenylenrest bedeutet, R für einen gegebenenfalls durch einen $Ph_1$-Rest substituierten niederaliphatischen Kohlenwasserstoffrest, wobei $Ph_1$ ein gegebenenfalls substituierter Phenylrest ist, einen 3 bis 7 Kohlenstoffatome enthaltenden Cycloalkylrest, einen durch einen heterocyclischen Rest aromatischen Charakters substituierten Niederalkylrest oder einen Rest der Formel $(CH_2CH_2O)_n-CH_2CH_2OR_4$ steht, worin n 0, 1 oder 2 ist und $R_4$ Wasserstoff oder Niederalkyl bedeutet, jedes der Symbole $R_1$ und $R_2$ für Wasserstoff oder Niederalkyl steht, $R_3$ einen gegebenenfalls substituierten Phenylrest $Ph_1$, einen gegebenenfalls durch $Ph_1$ substituierten Niederalkylrest, einen 3 bis 7 Kohlenstoffatome enthaltenden Cycloalkylrest, einen Cycloalkyl-$Ph_1$-Rest, worin Cycloalkyl und $Ph_1$ die oben angegebenen Bedeutungen haben, oder einen heterocyclischen Rest aromatischen Charakters bedeutet, und therapeutisch verwendbare Salze dieser Verbindung, zusammen mit einem pharmazeutischen Trägermaterial.

2. Neue substituierte Aether von 5-Hydroxy-5H-[1]benzopyrano[4,3-d]-pyrimidinen der allgemeinen Formel I, worin Ph einen gegebenenfalls substituierten 1,2-Phenylenrest bedeutet, R für einen gegebenenfalls durch einen $Ph_1$-Rest substituierten niederaliphatischen Kohlenwasserstoffrest, wobei $Ph_1$ ein gegebenenfalls substituierter Phenylrest ist,

einen 3 bis 7 Kohlenstoffatome enthaltenden Cycloalkylrest, einen durch einen heterocyclischen Rest aromatischen Charakters substituierten Niederalkylrest oder einen Rest der Formel $(CH_2CH_2O)_n-CH_2CH_2OR_4$ steht, worin n 0, 1 oder 2 ist und $R_4$ Wasserstoff oder Niederalkyl bedeutet, jedes der Symbole $R_1$ und $R_2$ für Wasserstoff oder Niederalkyl steht, $R_3$ einen gegebenenfalls substituierten Phenylrest $Ph_1$, einen gegebenenfalls durch $Ph_1$ substituierten Niederalkylrest, einen 3 bis 7 Kohlenstoffatome enthaltenden Cycloalkylrest, einen Cycloalkyl-$Ph_1$-Rest, worin Cycloalkyl und $Ph_1$ die oben angegebenen Bedeutungen haben, oder einen heterocyclischen Rest aromatischen Charakters bedeutet, und ihre Salze, mit Ausnahme der Verbindung der Formel I, worin Ph unsubstituiertes 1,2-Phenylen bedeutet, R für Aethyl steht, jedes der Symbole $R_1$ und $R_3$ Methyl bedeutet, $R_2$ für Wasserstoff steht und ihres Perchlorats.

3. Verbindungen der Formel I, worin Ph einen 1,2-Phenylenrest bedeutet, der gegebenenfalls durch Hydroxy, Niederalkyl, Hydroxy-niederalkyl, Niederalkoxy, Niederalkoxycarbonyl, Niederalkoxycarbonylniederalkyl, Halogen, Halogen-niederalkyl, Trifluormethyl, Nitro oder Amino substituiert sein kann, R für Niederalkyl, Niederalkenyl, Niederalkinyl, Cycloalkyl mit 3 bis 7 Kohlenstoffatomen, $Ph_1$-Niederalkyl, worin der Phenylrest $Ph_1$ analog dem oben genannten Rest Ph substituiert sein kann, oder einen durch einen Pyrryl-, Pyridyl - oder Thienylrest substituierten Niederalkylrest steht; jedes der Symbole $R_1$ und $R_2$ Wasserstoff oder Niederalkyl bedeutet, $R_3$ für einen oben definierten Phenylrest $Ph_1$, einen gegebenenfalls durch einen solchen Phenylrest $Ph_1$ substituierten Niederalkylrest oder für einen Pyrryl-, Pyridyl- oder Thienylrest steht, und ihre Salze, mit Ausnahme der Verbindung der Formel I, worin Ph unsubstituiertes 1,2-Phenylen bedeutet, R für Aethyl steht, jedes der Symbole $R_1$ und $R_3$ Methyl bedeutet, $R_2$ für Wasserstoff steht, und ihres Perchlorats.

4. Verbindungen der Formel I, worin Ph den 1,2-Phenylenrest bedeutet, R für Niederalkyl oder Phenyl-niederalkyl steht, jedes der Symbole $R_1$ und $R_2$ Wasserstoff bedeutet, $R_3$ für Niederalkyl, Phenyl, Niederalkoxy-phenyl, Phenyl-niederalkyl oder Pyridyl steht, und ihre Salze.

5. 2-(4-Methoxy-phenyl)-5-propyloxy-5H-[1]benzopyrano[4,3-d]pyrimidin und seine Salze.

6. Pharmazeutische Präparate enthaltend Verbindungen der im Anspruch 1 gezeigten Formel I, worin alle Symbole die im Anspruch 3 angegebenen Bedeutungen haben, und ihre Salze, mit Ausnahme der Verbindung der Formel I, worin Ph unsubstituiertes 1,2-Phenylen bedeutet, R für Aethyl steht, jedes der Symbole $R_1$ und $R_3$ Methyl bedeutet, $R_2$ für Wasserstoff steht, und ihres Perchlorats; zusammen mit einem pharmazeutischen Trägermaterial.

7. Die im Anspruch 1 genannten Verbindungen zur Anwendung in einem Verfahren zur therapeutischen Behandlung des menschlichen oder tierischen Körpers.

8. Die im Anspruch 3 genannten Verbindungen zur Anwendung in einem Verfahren zur therapeutischen Behandlung des menschlichen oder tierischen Körpers.

9. Die im Anspruch 1 genannten Verbindungen als lipidsenkende, antiarteriosklerotische und analgetische Mittel.

10. Die im Anspruch 3 genannten Verbindungen als lipidsenkende, antiarteriosklerotische und analgetische Mittel.

11. Verwendung der im Anspruch 1 genannten Verbindungen zur Herstellung von pharmazeutischen Präparaten.

12. Verwendung der im Anspruch 3 genannten Verbindungen zur Herstellung von pharmazeutischen Präparaten.

13. Verfahren zur Herstellung von neuen substituierten Aethern von 5-Hydroxy-5H-[1]benzopyrano[4,3-d]pyrimidinen der allgemeinen Formel I

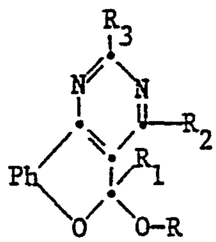

(I) ,

worin Ph einen gegebenenfalls substituierten 1,2-Phenylenrest bedeutet, R für einen gegebenenfalls durch einen $Ph_1$-Rest substituierten niederaliphatischen Kohlenwasserstoffrest, wobei $Ph_1$ ein gegebenenfalls substituierter Phenylrest ist, einen 3 bis 7 Kohlenstoffatome enthaltenden Cycloalkylrest, einen durch einen heterocyclischen Rest aromatischen Charakters substituierten Niederalkylrest oder einen Rest der Formel $(CH_2CH_2O)_n-CH_2CH_2OR_4$ steht, worin n 0, 1 oder 2 ist und $R_4$ Wasserstoff oder Niederalkyl bedeutet, jedes der Symbole $R_1$ und $R_2$ für Wasserstoff oder Niederalkyl steht, $R_3$ einen gegebenenfalls substituierten Phenylrest $Ph_1$, einen gegebenenfalls durch $Ph_1$ substituierten Niederalkylrest, einen 3 bis 7 Kohlenstoffatome enthaltenden Cycloalkylrest, einen Cycloalkyl-$Ph_1$-Rest, worin Cycloalkyl und $Ph_1$ die oben angegebenen Bedeutungen haben, oder einen heterocyclischen Rest aromatischen Charakters bedeutet, und ihren Salzen, mit Ausnahme der Verbindung der Formel I, worin Ph unsubstituiertes 1,2-Phenylen bedeutet, R für Aethyl steht, jedes der Symbole $R_1$ und $R_3$ Methyl bedeutet, $R_2$ für Wasserstoff steht, und ihres Perchlorats, dadurch gekennzeichnet, dass man

a) eine Verbindung der allgemeinen Formel II mit einer Verbindung der allgemeinen Formel III

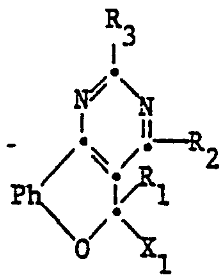 mit $X_2$-R (III)

(II)

veräthert, worin einer der Reste $X_1$ und $X_2$ eine freie, metallisierte oder reaktionsfähige veresterte Hydroxygruppe bedeutet und der andere für eine freie, reaktionsfähige veresterte bzw. metallisierte Hydroxygruppe steht, oder $X_2$ gemeinsam mit dem Rest R in der Verbindung $X_2$-R eine den Rest R einführende Verbindung darstellt, wenn $X_1$ eine freie Hydroxygruppe bedeutet, oder

b) eine Verbindung der allgemeinen Formel IV

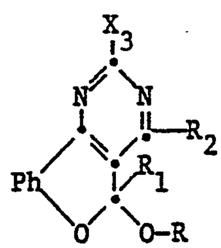

(IV) ,

worin $X_3$ eine abspaltbare Gruppe bedeutet, mit einer metallorganischen Verbindung der Formel $R_3$-M, worin M für eine metallische Gruppierung steht, oder mit einem $R_3$-Halogenid in Anwesenheit eines Metalls, kondensiert, und, wenn erwünscht, eine erhaltene Verbindung der Formel I in eine andere erfindungsgemässe Verbindung umwandelt, und/oder, wenn erwünscht, eine erhaltene freie Verbindung in ein Salz oder ein Salz in die freie Verbindung oder in ein anderes Salz überführt, und/oder wenn erwünscht, ein erhaltenes Gemisch von Isomeren oder Razematen in die einzelnen Isomeren oder Razemate auftrennt, und/oder, wenn erwünscht, erhaltene Razemate in die optischen Antiposen aufspaltet.

14. Die nach dem Verfahren des Anspruchs 13 erhältlichen Verbindungen.

Patentansprüche

(für Oesterreich)

1. Verfahren zur Herstellung von neuen substituierten Aethern von 5-Hydroxy-5H-[1]benzopyrano]4,3-d]pyrimidinen der allgemeinen Formel I

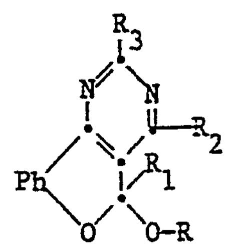

(I) ,

worin Ph einen gegebenenfalls substituierten 1,2-Phenylenrest bedeutet, R für einen gegebenenfalls durch einen $Ph_1$-Rest substituierten niederaliphatischen Kohlenwasserstoffrest, wobei $Ph_1$ ein gegebenenfalls substituierter Phenylrest ist, einen 3 bis 7 Kohlenstoffatome enthaltenden Cycloalkylrest, einen durch einen heterocyclischen Rest aromatischen Charakters substituierten Niederalkylrest oder einen Rest der Formel $(CH_2CH_2O)_n-CH_2CH_2OR_4$ steht, worin n 0, 1 oder 2 ist und $R_4$ Wasserstoff oder Niederalkyl bedeutet, jedes der Symbole $R_1$ und $R_2$ für Wasserstoff oder Niederalkyl steht, $R_3$ einen gegebenenfalls substituierten Phenylrest $Ph_1$, einen gegebenenfalls durch $Ph_1$ substituierten Niederalkylrest, einen 3 bis 7 Kohlenstoffatome enthaltenden Cycloalkylrest, einen Cycloalkyl-$Ph_1$-Rest, worin Cycloalkyl und $Ph_1$ die oben angegebenen Bedeutungen haben, oder einen heterocyclischen Rest aromatischen Charakters bedeutet, und ihren Salzen, mit Ausnahme der Verbindung der Formel I, worin Ph unsubstituiertes 1,2-Phenylen bedeutet, R für Aethyl steht, jedes der Symbole $R_1$ und $R_3$ Methyl bedeutet, $R_2$ für Wasserstoff steht, und ihres Perchlorats, dadurch gekennzeichnet, dass man

a) eine Verbindung der allgemeinen Formel II mit einer Verbindung der allgemeinen Formel III

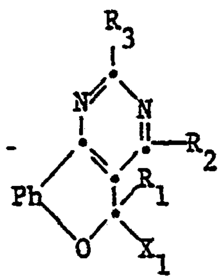

(II) mit $X_2$-R (III)

veräthert, worin einer der Reste $X_1$ und $X_2$ eine freie, metallisierte oder reaktionsfähige veresterte Hydroxygruppe bedeutet und der andere für eine freie, reaktionsfähige veresterte bzw. metallisierte Hydroxygruppe steht, oder $X_2$ gemeinsam mit dem Rest R in der Verbindung $X_2$-R eine den Rest R einführende Verbindung darstellt, wenn $X_1$ eine freie Hydroxygruppe bedeutet, oder

b) eine Verbindung der allgemeinen Formel IV

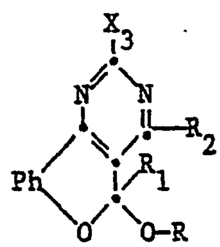

(IV) ,

worin $X_3$ eine abspaltbare Gruppe bedeutet, mit einer metallorganischen Verbindung der Formel $R_3$-M, worin M für eine metallische Gruppierung steht, oder mit einem $R_3$-Halogenid in Anwesenheit eines Metalls, kondensiert, und, wenn erwünscht, eine erhaltene Verbindung der Formel I in eine andere erfindungsgemässe Verbindung umwandelt, und/oder, wenn erwünscht, eine erhaltene freie Verbindung in ein Salz oder ein Salz in die freie Verbindung oder in ein anderes Salz überführt, und/oder wenn erwünscht, ein erhaltenes Gemisch von Isomeren oder Razematen in die einzelnen Isomeren oder Razemate auftrennt, und/oder, wenn erwünscht, erhaltene Razemate in die optischen Antiposen aufspaltet.

2. Verfahren nach Anspruch 1, dadurch gekennzeichnet, dass man in der Reaktion a) als die den Rest R einführende Verbindung $X_2$-R eine entsprechende Diazoverbindung, ein dem Alkohol ROH entsprechendes Acetal, einen entsprechenden Orthoester, ein entsprechendes Oxonium-, Carbenium- oder Haloniumsalz oder eine entsprechende Triazenverbindung verwendet.

3. Verfahren nach Anspruch 1, dadurch gekennzeichnet, dass man in der Reaktion b) Ausgangsstoffe der Formel IV verwendet, worin die abspaltbare Gruppe $X_3$ den Rest einer starken anorganischen Säure oder einer organischen Sulfonsäure bedeutet.

4. Verfahren nach Anspruch 1, dadurch gekennzeichnet, dass man Verbindungen der Formel I, worin Ph einen 1,2-Phenylenrest bedeutet, der gegebenenfalls durch Hydroxy, Niederalkyl, Hydroxy-niederalkyl, Niederalkoxy, Niederalkoxycarbonyl, Niederalkoxycarbonylniederalkyl, Halogen, Halogen-niederalkyl, Trifluormethyl, Nitro oder Amino substituiert sein kann, R für Niederalkyl, Niederalkenyl, Niederalkinyl, Cycloalkyl mit 3 bis 7 Kohlenstoffatomen, $Ph_1$-Niederalkyl, worin der Phenylrest $Ph_1$ analog dem oben genannten Rest Ph substituiert sein kann, oder einen durch einen Pyrryl-, Pyridyl - oder Thienylrest substituierten Niederalkylrest steht; jedes der Symbole $R_1$ und $R_2$ Wasserstoff oder Niederalkyl bedeutet, $R_3$ für einen oben definierten Phenylrest $Ph_1$, einen gegebenenfalls durch einen solchen Phenylrest $Ph_1$ substituierten Niederalkylrest oder für einen Pyrryl-, Pyridyl- oder Thienylrest steht, und ihre Salze, mit Ausnahme der Verbindung der Formel I, worin Ph unsubstituiertes 1,2-Phenylen bedeutet, R für Aethyl steht, jedes der Symbole $R_1$ und $R_3$ Methyl bedeutet, $R_2$ für Wasserstoff steht, und ihres Perchlorats, herstellt.

5. Verfahren nach Anspruch 1, dadurch gekennzeichnet, dass man Verbindungen der Formel I, worin Ph einen 1,2-Phenylenrest bedeutet, der gegebenenfalls durch Hydroxy, Niederalkyl, Niederalkoxy, Niederalkoxycarbonylniederalkyl, Halogen oder Trifluormethyl substituiert sein kann, R für Niederalkyl oder $Ph_1$-Niederalkyl steht, worin der Phenylrest $Ph_1$ analog dem oben genannten Rest Ph substituiert sein kann, jedes der Symbole $R_1$ und $R_2$ Wasserstoff oder Niederalkyl bedeutet, $R_3$ für einen wie oben definierten Phenylrest $Ph_1$, einen gegebenenfalls durch einen solchen Phenylrest $Ph_1$ substituierten Niederalkylrest oder für Pyridyl steht, und ihre Salze, mit Ausnahme der Verbindung der Formel I, worin Ph unsubstituiertes 1,2-Phenylen bedeutet, R für Aethyl steht, jedes der Symbole $R_1$ und $R_3$ Methyl bedeutet, $R_2$ für Wasserstoff steht, und ihres Perchlorats, herstellt.

6. Verfahren nach Anspruch 1, dadurch gekennzeichnet, dass man Verbindungen der Formel I, worin Ph den 1,2-Phenylenrest bedeutet, R für Niederalkyl oder Phenyl-niederalkyl steht, jedes der Symbole $R_1$ und $R_2$ Wasserstoff bedeutet, $R_3$ für Niederalkyl, Phenyl, Niederalkoxy-phenyl, Phenyl-niederalkyl oder Pyridyl steht, und ihre Salze, herstellt.

7. Verfahren nach Anspruch 1, dadurch gekennzeichnet, dass man Verbindungen der Formel I, worin Ph den 1,2-Phenylenrest bedeutet, R für Niederalkyl steht, jedes der Symbole $R_1$ und $R_2$ Wasserstoff bedeutet, $R_3$ für den gegebenenfalls durch Niederalkoxy substituierten Phenylrest steht, und ihre Salze, herstellt.

8. Verfahren nach Anspruch 1, dadurch gekennzeichnet, dass man 2-(4-Methoxy-phenyl)-5-propyloxy-5H-[1]benzopyrano[4,3-d]pyrimidin und seine Salze herstellt.

9. Verfahren nach Anspruch 1 zur Herstellung von neuen substituierten Aethern von 5-Hydroxy-5H-[1]benzopyrano[4,3-d]pyrimidinen der im Anspruch 1 gezeigten allgemeinen Formel I, worin alle Symbole die im Anspruch 1 angegebenen Bedeutungen haben, und ihren Salzen, mit

Ausnahme der Verbindung der Formel I, worin Ph unsubstituiertes 1,2-Phenylen bedeutet, R für Aethyl steht, jedes der Symbole $R_1$ und $R_3$ Methyl bedeutet, $R_2$ für Wasserstoff steht, und ihres Perchlorats, dadurch gekennzeichnet, dass man

a) eine Verbindung der allgemeinen Formel II mit einer Verbindung der allgemeinen Formel III

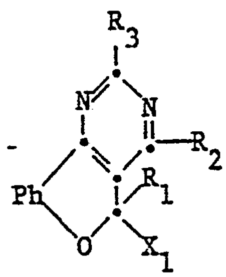

(II) mit $X_2$-R (III)

veräthert, worin einer der Reste $X_1$ und $X_2$ eine freie, metallisierte oder reaktionsfähige veresterte Hydroxygruppe bedeutet und der andere für eine freie, reaktionsfähige veresterte bzw. metallisierte Hydroxygruppe steht, oder $X_2$ gemeinsam mit dem Rest R in der Verbindung $X_2$-R eine den Rest R einführende Verbindung darstellt, wenn $X_1$ eine freie Hydroxygruppe bedeutet, oder

b) eine Verbindung der allgemeinen Formel IV

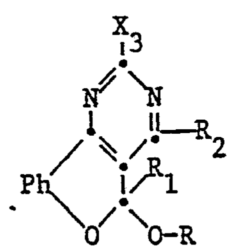

(IV) ,

worin $X_3$ ein Halogenatom bedeutet, mit einer metallorganischen Verbindung der Formel $R_3$-M, worin M für eine metallische Gruppierung steht, oder mit einem $R_3$-Halogenid in Anwesenheit eines Metalls, kondensiert, und, wenn erwünscht, eine erhaltene Verbindung der Formel I in eine andere erfindungsgemässe Verbindung umwandelt, und/oder, wenn erwünscht, eine erhaltene freie Verbindung in ein Salz oder ein

Salz in die freie Verbindung oder in ein anderes Salz überführt, und/oder wenn erwünscht, ein erhaltenes Gemisch von Isomeren oder Razematen in die einzelnen Isomeren oder Razemate auftrennt, und/oder, wenn erwünscht, erhaltene Razemate in die optischen Antiposen aufspaltet.

10. Verfahren nach Anspruch 9, dadurch gekennzeichnet, dass man Verbindungen der Formel I, worin Ph einen 1,2-Phenylenrest bedeutet, der gegebenenfalls durch Hydroxy, Niederalkyl, Niederalkoxy, Niederalkoxycarbonylniederalkyl, Halogen oder Trifluormethyl substituiert sein kann, R für Niederalkyl oder $Ph_1$-Niederalkyl steht, worin der Phenylrest $Ph_1$ analog dem oben genannten Rest Ph substituiert sein kann, jedes der Symbole $R_1$ und $R_2$ Wasserstoff oder Niederalkyl bedeutet, $R_3$ für einen wie oben definierten Phenylrest $Ph_1$, einen gegebenenfalls durch einen solchen Phenylrest $Ph_1$ substituierten Niederalkylrest oder für Pyridyl steht, und ihre Salze, mit Ausnahme der Verbindung der Formel I, worin Ph unsubstituiertes 1,2-Phenylen bedeutet, R für Aethyl steht, jedes der Symbole $R_1$ und $R_3$ Methyl bedeutet, $R_2$ für Wasserstoff steht, und ihres Perchlorats, herstellt.

11. Verfahren nach Anspruch 9, dadurch gekennzeichnet, dass man Verbindungen der Formel I, worin Ph den 1,2-Phenylenrest bedeutet, R für Niederalkyl steht, jedes der Symbole $R_1$ und $R_2$ Wasserstoff bedeutet, $R_3$ für den gegebenenfalls durch Niederalkoxy substituierten Phenylrest steht, und ihre Salze, herstellt.

12. Verfahren nach Anspruch 9, dadurch gekennzeichnet, dass man 2-(4-Methoxy-phenyl)-5-propyloxy-5H-[1]benzopyrano[4,3-d]pyrimidin und seine Salze herstellt.

13. Verfahren zur Herstellung eines pharmazeutischen Präparates, gekennzeichnet durch die Verarbeitung eines Wirkstoffes der im Anpsruch 1 gezeigten Formel I, worin Ph einen gegebenenfalls substituierten

1,2-Phenylenrest bedeutet, R für einen gegebenenfalls durch einen $Ph_1$-Rest substituierten niederaliphatischen Kohlenwasserstoffrest, wobei $Ph_1$ ein gegebenenfalls substituierter Phenylrest ist, einen 3 bis 7 Kohlenstoffatome enthaltenden Cycloalkylrest, einen durch einen heterocyclischen Rest aromatischen Charakters substituierten Niederalkylrest oder einen Rest der Formel $(CH_2CH_2O)_n-CH_2CH_2OR_4$ steht, worin n 0, 1 oder 2 ist und $R_4$ Wasserstoff oder Niederalkyl bedeutet, jedes der Symbole $R_1$ und $R_2$ für Wasserstoff oder Niederalkyl steht, $R_3$ einen gegebenenfalls substituierten Phenylrest $Ph_1$, einen gegebenenfalls durch $Ph_1$ substituierten Niederalkylrest, einen 3 bis 7 Kohlenstoffatome enthaltenden Cycloalkylrest, einen Cycloalkyl-$Ph_1$-Rest, worin Cycloalkyl und die oben angegebenen Bedeutungen haben, oder einen heterocyclischen Rest aromatischen Charakters bedeutet, oder eines therapeutisch verwendbaren Salzes davon, mit einem pharmazeutischen Trägermaterial.

14. Verfahren zur Herstellung eines pharmazeutischen Präparates, gekennzeichnet durch die Verarbeitung eines erfindungsgemässen Wirkstoffes nach dem Anspruch 4, mit einem pharmazeutischen Trägermaterial.